(19) Europäisches Patentamt
European Patent Office
Office européen des brevets

(11) Publication number: **0 004 766**
**A2**

(12) # EUROPEAN PATENT APPLICATION

(21) Application number: 79300542.2

(22) Date of filing: 02.04.79

(51) Int. Cl.$^2$: C 07 J 5/00
C 07 J 7/00, C 07 J 71/00
A 61 K 31/57, A 61 K 31/58
//C07J21/00

(30) Priority: 05.04.78 US 893641

(43) Date of publication of application: 17.10.79 Bulletin 79/21

(84) Designated Contracting States: BE CH DE FR GB NL SE

(71) Applicant: SYNTEX (U.S.A.) INC.
3401 Hillview Avenue
Palo Alto, California 94304(US)

(72) Inventor: Alvarez, Francisco S.
1089 Rochester Court
Sunnyvale California 94087(US)

(74) Representative: Armitage, Ian Michael et al,
MEWBURN ELLIS & CO. 70/72 Chancery Lane
London WC2A 1AD(GB)

(54) **4-Halo-pregn-4-enes, their preparation and pharmaceutical use.**

(57) 4-Halo-pregn-4-enes of the formula:

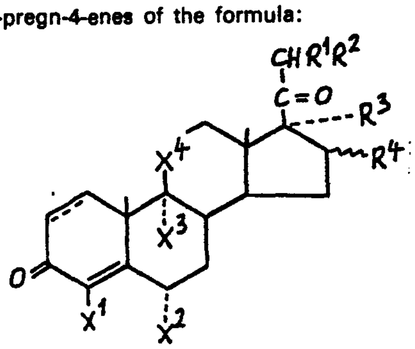

(I)

wherein

$X^1$ is fluoro or chloro;

$X^2$ is hydrogen, fluoro or chloro;

$X^3$ is hydrogen, fluoro, chloro or bromo;

$X^4$ is =C=O or 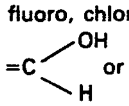 or may be

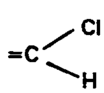 when $X^3$ is chloro;

$R^1$ is hydrogen, fluoro, chloro, bromo, hydroxy or methoxy;

$R^2$ is fluoro, chloro, hydroxy or alkanoyloxy of 2 to 6 carbon atoms when $R^1$ is hydrogen or $R^2$ is the same as $R^1$ when $R^1$ is fluoro, chloro, hydroxy or methoxy;

$R^3$ is hydroxy or alkanoyloxy of 2 to 6 carbon atoms when $R^4$ is α-methyl or β-methyl or $R^3$ and $R^4$ together are

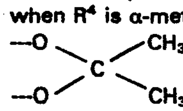 ; and

the solid and broken lines between the 1- and 2-positions in the A ring of the steroid nucleus represents a single or a double bond;

have topical anti-inflammatory activity in mammals. They can be prepared by contacting the corresponding $\Delta^5$ compound with a base. The 17α-alkanoyloxy compound can be prepared by esterifying the corresponding 17α-hydroxy compound.

EP 0 004 766 A2

# 4-HALO-PREGN-4-ENES, THEIR PREPARATION AND PHARMACEUTICAL USE

This invention relates to corticosteroids. More specifically it relates to pregn-4-enes which are substituted at the 4 position with fluoro or chloro and which are topically active anti-inflammatory agents in mammals. The invention further relates to pharmaceutically active compositions comprising the compound of the invention and to a process for preparing the compounds of the invention.

4-Chlorohydrocortisone and 4-fluorohydrocortisone are known compounds. However, no biological activity is shown for these compounds. See U.S. 3,232,960 to Magerlein and Ernst Jucher, Process in Drug Research, Vol. 5, p. 50, Birkhauser Verlag Basel and Stuttart, 1963.

U.S. 3,192,301 to Westerhof discloses a process for making certain steroids. At column 3 of the patent the broad formula indicates that the patent relates to compounds of the general formula

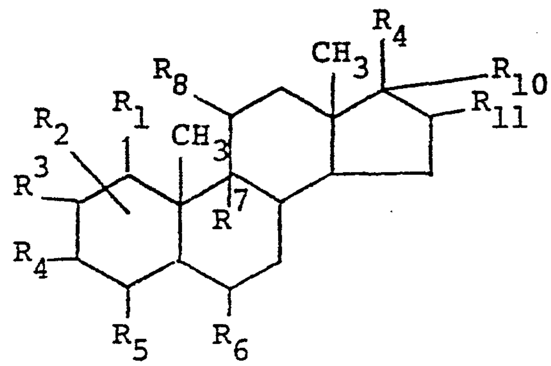

wherein $R^5$ and $R^6$ may be, i.a., halogen. However, the patent does not disclose the preparation of any 4,6α-dihalo steroids.

U.S. Patent 3,707,537 to Kierstead discloses certain 4-chloro-6-halo-pregna-4,6-dienes which require the presence of a 6-7 double bond. Another patent which discloses the possibility of certain 4,6-dihalo-pregna-4-enes is U.S. 3,489,748 to Benningen and Muller. These compounds differ from the compounds of this invention in that the Benningen et al compounds exhibit a 16α-alkylthio group at the 16 position and have no substitution at the 17α position. Furthermore, there are no di-substituted compounds disclosed, only 4-chloro-pregna-4,6,16-triene-3,20-dione and the corresponding 16α-ethylthiopregna-4,6-diene.

Other 3-keto-$\Delta^4$ steroids are also known which are 6,6-difluoro substituted. See, for example, U.S. 3,219,673; U.S. 3,767,684; U.S. 3,822,253; U.S. 3,681,338; U.S. 3,718,673; U.S. 3,546,215; and U.S. 3,629,242.

A heretofore unknown series of compounds has now been disclovered which show high topical anti-inflammatory activity.

One aspect of this invention is a compound chosen from those represented by the formula

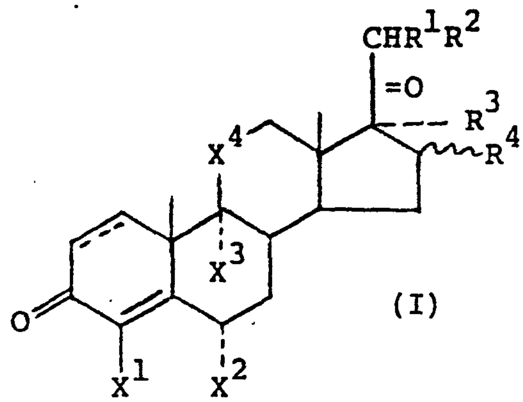

(I)

wherein

$X^1$ is fluoro or chloro;

$X^2$ is hydrogen, fluoro or chloro;

$X^3$ is hydrogen, fluoro, chloro or bromo;

$X^4$ is =C=O or 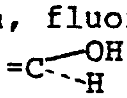 or may be 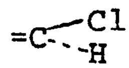 when $X^3$ is chloro;

$R^1$ is hydrogen, fluoro, chloro, hydroxy or methoxy;

$R^2$ is fluoro, chloro, bromo, hydroxy or alkanoyloxy of 2 to 6 carbon atoms when $R^1$ is hydrogen or $R^2$ is the same as $R^1$ when $R^1$ is fluoro, chloro, hydroxy or methoxy;

$R^3$ is hydroxy or alkanoyloxy of 2 to 6 carbon atoms when $R^4$ is α-methyl or β-methyl or $R^3$ and $R^4$ together are 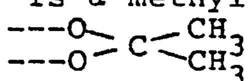 ; and

the solid and broken lines between the 1 and 2 positions in the A ring of the steroid nucleus represent a single or double bond.

Another aspect of this invention is a topical anti-inflammatory pharmaceutical composition which comprises at least one suitable pharmaceutical excipient in combination with a compound chosen from those represented by Formula (I), above, wherein each of the substituents are as defined. Particularly valuable compounds in this composition are set forth hereafter.

Still another aspect of this invention is the use of compounds of Formula (I) for treating an inflamed condition in a mammal.

Still another aspect of this invention is a process for the preparation of a compound chosen from those represented by Formula (I), above, wherein the solid and broken lines between C-1 and C-2 represent a double or single bond. This process comprises contacting a compound represented by the formula

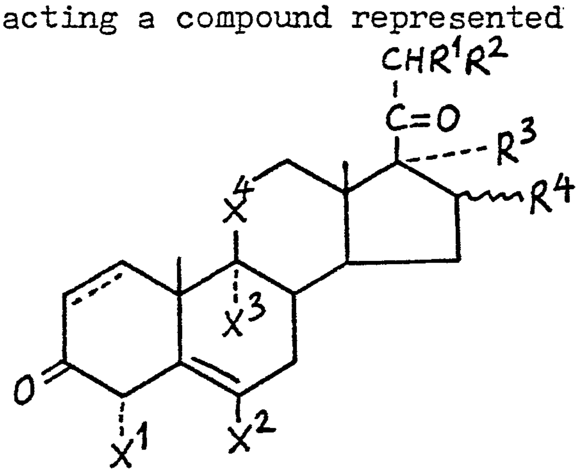

(IV)

with a base for a time sufficient to form a compound of the invention.

One subgroup of the broad aspect of this invention comprises those compounds represented by Formula (I) wherein $X^1$ is fluoro or chloro; $X^2$ is hydrogen or fluoro; $X^3$ is hydrogen or fluoro; $X^4$ is $-C{<}_{H}^{OH}$; $R^1$ is hydrogen, hydroxy,or methoxy; $R^2$ is the same as $R^1$ when $R^1$ is methoxy or hydroxy or is hydroxy, fluoro, chloro or acetoxy when $R^1$ is hydrogen; and $R^3$ and $R^4$ together are 16α,17α-isopropylidenedioxy.

Still another subgroup of this invention comprises the compounds represented by Formula (I) wherein $X^1$ is fluoro or chloro; $X^2$ is hydrogen or fluoro; $X^3$ is hydrogen or fluoro; $X^4$ is $=C{<}_{H}^{OH}$; $R^1$ is hydrogen, hydroxy or methoxy; $R^2$ is chloro, fluoro, hydroxy or acetoxy when $R^1$ is hydrogen or $R^2$ is the same as $R^1$ when $R^1$ is hydroxy or methoxy; $R^3$ is hydroxy or alkanoyloxy of 2-6 carbon atoms; and $R^4$ is α-methyl or β-methyl. A subdivision of this subgroup comprises the compounds represented by Formula (I) wherein $R^1$ is hydrogen and $R^2$ is fluoro, chloro, hydroxy or acetoxy; and $R^4$ is α-methyl.

Preferably, in each of the subgroups set forth hereinbefore there is a double bond between C-1 and C-2 of the steroid ring.

In naming the compounds of this invention the first substituent will be the 4-position and all other

substituents on the steroid ring shall be included numerically unless there is a substitutent the same as that at the 4-position. For example, where $X^1$ is chloro, $X^2$ and $X^3$ are fluoro, $X^4$ is hydroxy, $R^1$ is hydrogen, $R^2$ is methoxy, $R^3$ and $R^4$ together are 16α,17α-isopropylidenedioxy and there is a double bond between C-1 and C-2, the name is 4-chloro-6α,9α-difluoro-11β-hydroxy-16α,17α-isopropylidenedioxy-21-methoxypregna-1,4-diene-3,20-dione. If on the other hand, $R^3$ is hydroxy and $R^4$ is α-methyl while $R^1$, $R^2$ and $X^1$-$X^4$ are the same as immediately named above, the compound is named 4-chloro-6α,9α-difluoro-11β,17α-dihydroxy-16α-methyl-21-methoxypregna-1,4-diene-3,20-dione.

If, however, in the compound of Formula (I), $X^1$ and $X^3$ are both chloro, $X^4$ is $=C{<}^{Cl}_{H}$, $X^2$ is fluoro, $R^1$ is hydrogen, $R^2$ is chloro, $R^3$ and $R^4$ together are isopropylidenedioxy, and there is a double bond between C-1 and C-2, the compound is 4,9α,11β,21-tetrachloro-6α-fluoro-16α,17α-isopropylidenedioxypregna-1,4-diene-3,20-dione.

The compounds of this invention are useful for the relief of inflamed conditions in mammals, and more specifically are useful for relieving inflammatory manifestations of corticosteroid responsive dermatoses in humans. Initial approximation of anti-inflammatory activity is done by following the procedure of McKenzie, S.W. and Stoughton, R.B., "Method for Comparing Percutaneous Absorption of Steroids" Arch Dermat, 86, 608 (1962) or modifications thereof.

Generally, the inflammatory manifestation in mammals, particularly humans, is combatted by treating the afflicted mammal with a therapeutically effective amount of at least one of the novel steroids of this invention, that is an amount which results in improvement of the inflamed condition. Preferably the steroids are first formulated to prepare a suitable pharmaceutical formulation, as discussed hereinafter, which is then placed in contact with the afflicted area. A therapeutically effective amount will depend upon the particular condition and the mammal receiving the treatment but will vary between 0.001% to 10% by weight of the pharmaceutical composition and preferably will be between 0.01 and 1% by weight of the formulation. Using these levels in the formulation, a therapeutically effective, non-side effect producing amount, i.e. enough to effect an anti-inflammatory response, but not enough to adversely effect the recipient, is applied to the inflamed area.

The compounds of this invention not only have anti-inflammatory activity but also exhibit a low level of systemic activity, as measured by recognized laboratory assays. This allows for the application of an effective amount of the anti-inflammatory compounds with little adverse effect on the rest of the mammal's system.

The novel steroids of this invention may be formulated with suitable pharmaceutical excipients known in the art to form particularly effective anti-inflammatory compositions. Generally an effective amount of the steroid is about 0.001%w to about 10%w of the total formulated composition. The rest of the formulated composition will be about 90%w to about 99.999%w of suitable excipients which may include a pharmaceutically acceptable solvent and other pharmaceutically acceptable additives to form an effective pharmaceutical formulation.

A pharmaceutically acceptable solvent is one which is substantially non-toxic and non-irritating under the conditions used and may be readily formulated into any of the classical drug formulations such as powders, creams, ointments, lotions, gels, foams, suppositories,

aerosols, solutions or the like. Particularly suitable solvents include water, glycerine, propylene carbonate, and a glycol such as 1,2-propylene diol (i.e. propylene glycol), 1,3-propylene diol, polyethylene glycol having a molecular weight of from 100 to 10,000, dipropylene glycol, etc.; and mixtures of the aforementioned with each other.

A topical cream may be prepared as a semi-solid emulsion of oil in water or water in oil. A cream base formulation by definition is an emulsion which is a two-phase system with one liquid (for example fats or oils) being dispersed as small globules in another substance (e.g., a glycol-water solvent phase) which may be employed as the primary solvent for the novel steroids therein, the cream formulation may contain fatty alcohols, surfactants, mineral oil or petrolatum and other typical pharmaceutical adjuvants such as anti-oxidants, antiseptics, or compatible adjuvants. A typical cream base formulation is given in the following table:

| | |
|---|---|
| Water/glycol mixture (15% or more glycol) | 50 - 99 parts by weight |
| Fatty alcohol | 1 - 20 |
| Non-ionic Surfactant | 0 - 10 |
| Mineral oil | 0 - 10 |
| Typical pharmaceutical adjuvants | 0 - 5 |
| Active Ingredients | 0.001 - 10 |

The fatty alcohol, non-ionic surfactant, and other adjuvants are discussed in U.S. 3,934,013 to Poulsen which is incorporated herein by reference.

The novel steroids of this invention may also be formulated as ointments. A "classical" ointment is a semisolid anhydrous composition which may contain mineral oil, white petrolatum, a suitable solvent such

as a glycol and may include propylene carbonate and other pharmaceutically suitable additives such as surfactants, for example Span and Tween, or wool fat (lanolin), along with stabilizers such as antioxidants and other adjuvants as mentioned before. Following is an example of a typical "classical" ointment base:

| | |
|---|---|
| White petrolatum | 40 - 94 parts by weight |
| Mineral Oil | 5 - 20 |
| Glycol solvent | 1 - 15 |
| Surfactant | 0 - 10 |
| Stabilizer | 0 - 10 |
| Active Ingredients | 0.001 - 10.0 |

Other suitable ointment base formulations which employ propylene carbonate are described in U.S. patent 4,017,615 issued April 12, 1977 by Shastri et al entitled "Propylene Carbonate Ointment Vehicle" and U.S. 3,924,004 issued December 2, 1975 by Chang et al entitled "Fatty Alcohol-Propylene Carbonate-Glycol Solvent Cream Vehicle". As much of those applications as is pertinent is incorporated herein by reference. Following is a typical ointment base formulation containing propylene carbonate:

| | |
|---|---|
| Active Ingredients | 0.001 - 10.0 parts by weight |
| Propylene Carbonate | 1 - 10 |
| Solvent | 1 - 10 |
| Surfactant | 1 - 10 |
| White Petrolatum | 70 - 97 |

Suitable solvents, surfactants, stabilizers, etc. are discussed in U.S. 3,934,013 and such discussion is incorporated herein by reference.

A suitable "non-classical" anhydrous, water washable "ointment type" base is described in U.S. Patent No. 3,952,930 to Katz and Neiman, and that patent is incorporated herein by reference. A representative

composition of this invention utilizing such a base is as follows:

| | |
|---|---|
| Glycol solvent | 40 - 35 parts by weight |
| Fatty alcohol | 15 - 45 |
| Compatible plasticizer | 0 - 15 |
| Compatible coupling Agent | 0 - 15 |
| Penetrant | 0 - 20 |
| Active Ingredients | 0.001 - 10.0 |

The novel compounds of this invention are ultimately obtained by converting a compound represented by Formula (IV) to the corresponding compound of this invention represented by Formula (I) wherein $X^1$, $X^2$, $X^3$, $X^4$, $R^1$, $R^2$, $R^3$ and $R^4$ are defined hereinbefore.

The overall process for preparing the pregna-1,4-dienes compounds of the invention is a three step process which is illustrated by the following reaction sequence, wherein $X^3$ is chloro, fluoro or bromo and the other substituents are defined hereinbefore for the broadest aspect of the invention.

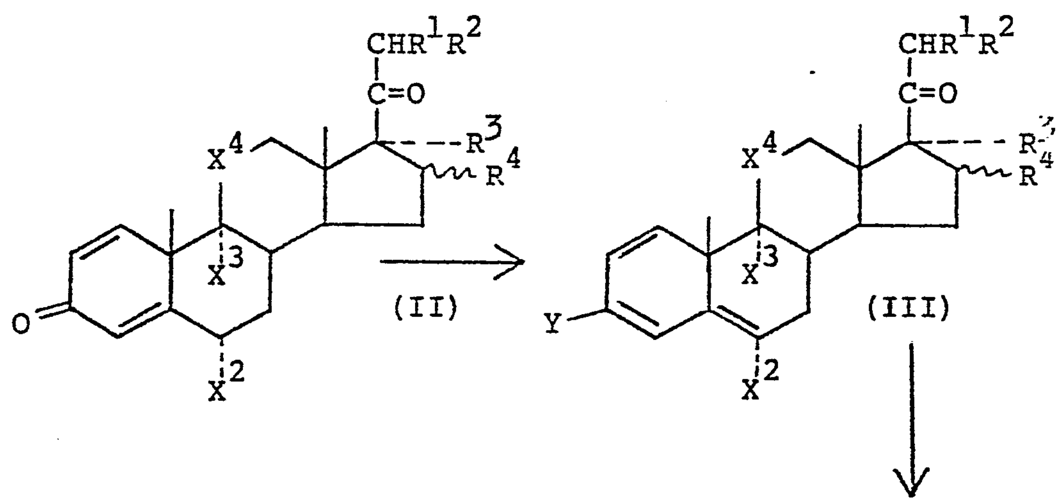

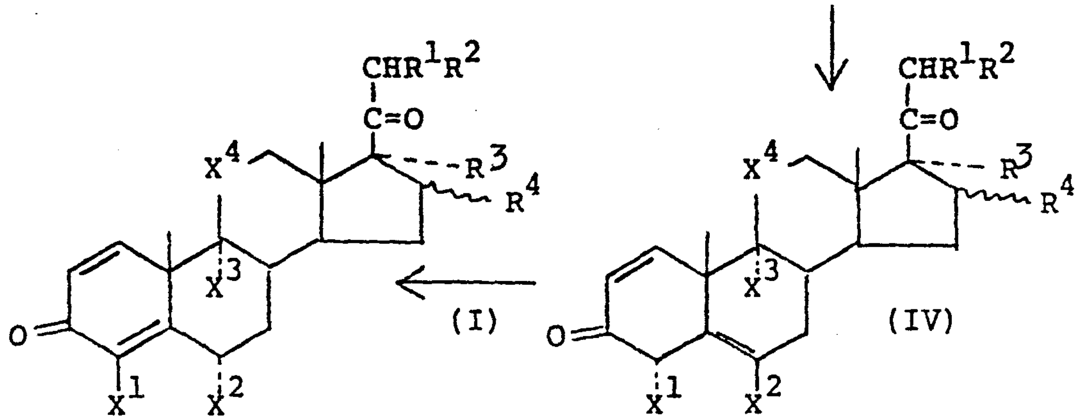

(I) (IV)

In the first step a compound represented by formula (II) is reacted to form a compound of the formula (III) wherein Y is methoxy or ethoxy. If, in the starting compound represented by formula (II), $R^2$ is hydroxy and $R^1$ is hydrogen then $R^2$ should be protected by acetylation or some similar means. If there is a β-hydroxy at the 11 position it is preferable that this hydroxy is also protected by acetylation or the like. Protection of both positions is readily accomplished by reacting the compound represented by (II) with acetic anhydride in pyridine and triethylamine in the preence of catalytic amounts of dimethyl pyridine at about 10° to about 100°C, preferably at room temperature, to give a compound of formula (IIa)

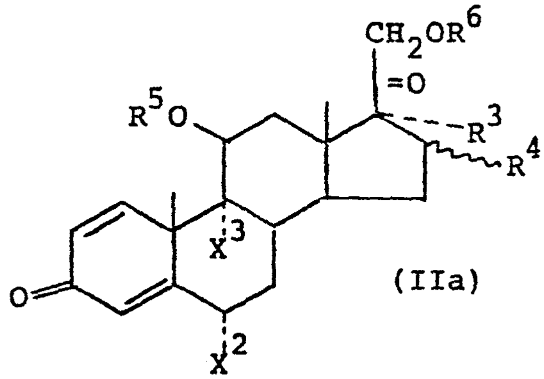

(IIa)

where $R^5$ and $R^6$ are each $CH_3-\overset{O}{\overset{\|}{C}}-$. Although such protection is not needed in the case of the 11β-hydroxy, it does improve the yield.

If compound (II) is a 17α,21-dihydroxypregna-1,4-diene where $R^4$ is methyl or hydrogen, it is preferably reacted with acid aqueous formaldehyde to form the 17α,21:20,21-bis methylenedioxy compound of the formula

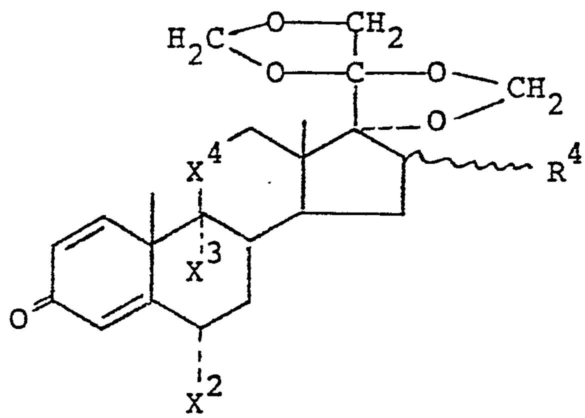

(IIb)

Thus both the 17 and 21 hydroxy moieties are protected from reaction. Once the hydroxy groups, if any, are protected, compound (II), (IIa) or (IIb) is reacted with, for example, a large excess of trimethyl orthoformate in methanol or triethyl orthoformate in ethanol in the presence of a catalytic amount of a suitable acid catalyst, such as fuming sulfuric acid, at reflux temperature or less. About 50-55°C is preferred. Generally the molar ratio of trimethyl orthoformate or triethyl orthoformate to the compound represented by formula (II), (IIa) or (IIb) is about 10:1 to about 30:1, preferably about 10:1 to 15:1. Once the reaction is complete a base is added to neutralize the acid and the resulting enol-ether represented by formula (III) or the enol-ether corresponding to the compound represented by formulas (IIa) or (IIb) where Y is methoxy or ethoxy is recovered and purified using methods well known in the art such as evaporation, recrystallization, etc.

The enol-ether is then fluorinated or chlorinated using perchloryl fluoride ($ClO_3F$) or trifluoromethoxy

fluoride ($CF_3OF$) as a fluorinating agent, a source of positive chlorine such as N-chlorosuccinimide, dichlorohydantoin, etc. as a chlorinating agent, to form the 3-keto-4α-fluoro (chloro) pregna-1,5-diene represented by formula (IV) or the 3-keto-4α-fluoro (chloro)-pregna-1,5-diene corresponding to the compound represented by formulas (IIa) or (IIb).

In the case of $ClO_3F$, which is a gas, an approximately equimolar amount (1 to 1.1 moles $ClO_3F$ per mole of the enol-ether) is metered in to a mixture of the enol-ether in a solution which is a major amount of acetone, preferably 90% by weight, and a minor amount of water, preferably about 10% over a period of about 1-3 hours at 10-30°C, preferably ambient temperature. Dichlorohydantoin is similarly reacted.

The compound of formula (IV), or the corresponding compound from formula (IIa) or (IIb), is reacted in an inert atmosphere, e.g. nitrogen, with a suitable base such as an alkali metal carbonate, e.g. potassium carbonate, in a suitable oxygenated hydrocarbon solvent such as an alkanol, e.g. methanol, to cause a rearrangement to take place to form the desired 4-fluoro (4-chloro)-3-keto pregna-1,4-diene.

If appropriate, the protecting groups at the 11β,17α- and/or 21-positions are hydrolyzed using a suitable base such as 1 molar sodium hydroxide if the treatment with, e.g. potassium carbonate is not sufficient. Where $X^3$ is fluoro, chloro or bromo, potassium carbonate is generally sufficient for hydrolysis of the 11β-acetoxy group. In the case of the 9α-chloro- or 9α-bromo-11β-hydroxy compound, the treatment with potassium carbonate or sodium hydroxide may form a 9,11-epoxide. This can be converted to the 9,11-chlorohydrin or 9,11-bromohydrin compound by reacting the 9,11-epoxide with hydrochloric or hydrobromic acid in chloroform respectively. The BMD compound is hydrolyzed using a suitable acid such as 60% formic acid or 48% hydrofluoric acid.

Once the final pregna-1,4-diene is obtained, the compound may be readily selectively hydrogenated across the 1-2 bond by any suitable means known in the art to obtain the corresponding $\Delta^4$-3-keto steroid.

To prepare the 4-halopregn-4-enes of formula (I) of this invention wherein $X^3$ is hydrogen, a slightly modified route is required as set forth in the following reaction sequence:

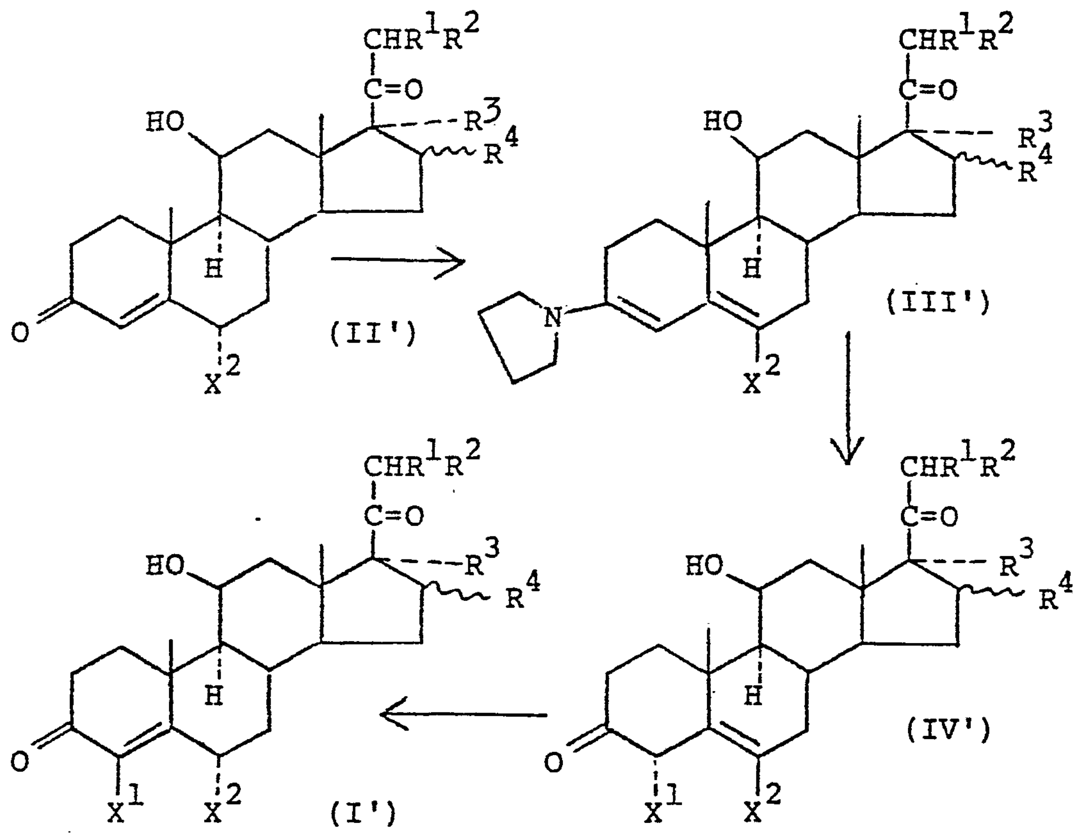

In this sequence, the starting 3-keto-pregn-4-ene represented by formula (II') is first reacted with a suitable amine such as pyrrolidine to form the enamine represented by Formula (III'). This reaction takes place readily at reflux temperatures in a suitable inert

organic solvent such as benzene or methanol.

The enamine represented by formula (III') is then fluorinated or chlorinated according to the procedure set forth above to form the 4α-fluoro (chloro)-3-keto-pregn-5-ene of formula (IV') which in turn is treated with, e.g. potassium carbonate in methanol under $N_2$, to shift the double bond to the 4-position thus giving a compound of this invention represented by (I'). The resulting 3-keto-pregn-4-ene is readily dehydrogenated at the 1-position by methods known in the art such as using 2,3-dichloro-4,6-dicyano-1,4-benzoquinone in dioxane. Any protecting groups at 11β-, 17α-, or 21 are readily hydrolyzed by methods discussed hereinbefore.

More specific embodiments of the process of this invention are given hereafter in the Examples.

Preparation Of Starting Compounds

As pointed out above, starting compounds for the process of preparing the compounds of this invention are represented by the formulas (II) or (II') wherein the substituents are described hereinbefore for each of the respective formulas and are either known per se or are readily prepared using known techniques. For example, pregna-1,4,9(11)-trienes are treated with chlorine according to the process of U.S. 3,009,933 to give the corresponding 9α,11β-dichloropregna-1,4-diene. The 9α-bromo-11β-hydroxy or 9α-chloro-11β-hydroxy compounds are prepared by reacting the appropriate pregna-1,4,9(11)-triene with dibromohydantoin or N,N'-dichloro-dimethylhydantoin to form the 9α,11β-bromohydrin or 9α,11β-chlorohydrin, respectively. The 9α,11β-bromohydrin steroid is in turn reacted with sodium hydroxide to give the corresponding 9β,11β-epoxide which is then treated with a hydrogen fluoride/urea complex according to the process set forth in U.S. 3,211,758 to Tarkoey to give the 9α-fluoro-11β-hydroxy compound.

An 11β-hydroxy (-unsubstituted) steroid is readily prepared from a 3-keto-6α-optionally substituted-pregna-1,4-diene or pregn-4-ene by methods well known in the art such as employing Cunninghamella blakesleeana, Cunninghamella bainieri, Curvularia Lunata, or other suitable micro-organisms in ethanol which selectively affords the desired 11β-hydroxy steroid.

16α,17α-Isopropylidenedioxy-pregna-1,4,9(11)-trienes are known or can readily be prepared by reacting an 11β-hydroxy-16α,17α-isopropylidenedioxypregna-1,4-diene with thionyl chloride in pyridine at 0°C. A 17α-hydroxypregna-1,4-9(11)-triene is prepared by reacting the corresponding 11β,17α-dihydroxy-pregna-1,4-diene with methane sulfonyl chloride in pyridine with $SO_3$ at about 0°C or with methyl chlorosulfinate (prepared by distilling methanol with thionylchloride) in tetrahydrofuran and pyridine at -78°C and allowing the reaction mixture to slowly warm to ambient temperature.

A 16α,17α-isopropylidenedioxy group is readily introduced by treating the corresponding 16α,17α-dihydroxy steroid with acetone in the presence of perchloric acid. The 16α,17α-dihydroxy groups are introduced by treating a pregn-16-ene derivative with potassium permanganate, acetone and acetic acid.

A 16-methyl group is introduced by treating the corresponding 20-keto-pregn-16-ene steroid with methyl magnesium bromide in the presence of cuprous chloride in an ether such as tetrahydrofuran. The 20-keto-pregn-16-ene steroid is prepared by preparing the 3,20-bis-semicarbazone of a 3,20-diketo-17α-hydroxy steroid, treating it with glacial acetic acid and acetic anhydride and then allowing the resulting product to react with aqueous pyruvic acid.

The 17α-hydroxy group is introduced in conjunction

with the 16-methyl group by first treating the corresponding 16-methyl-pregn-16-ene steroid (which is prepared by treating the corresponding pregn-16-ene steroid with diazomethane and then heating the resulting product to 180°C.) with hydrogen perioxide, in an aqueous basic media, then permitting the resulting 16,17-oxido-16-methyl steroid to react with hydrogen bronide in glacial acetic acid. The resulting olefin is hydrogenated with the use of a palladium catalyst to afford the corresponding 16α-methyl-17α-hydroxy derivative.

The 21-chloro, bromo or fluoro group is introduced by treating a 21-hydroxy steroid derivative with methanesulfonyl chloride in pyridine, then refluxing the resulting 21-mesylate ester with lithium chloride or lithium bromide in sulfolane at 135°C to get the corresponding 21-chloro or -bromo steroid derivative. The 21-fluoro steroid is obtained by refluxing the 21-bromide with potassium fluoride in sulfolane at 135°C. Preferably the 21-bromo compound is isolated before preparing the 21-fluoro compound. Further discussion of the preparation of these 21-halo-steroids can be found in U.S. 3,053,830 to Fried.

The 21-acyloxy compounds are readily prepared by reacting the known corresponding 21-hydroxy compounds, e.g. prednisolone, with the desired alkanoyl chloride or, preferably, anhydride and an organic base, e.g. pyridine.

The 21,21-dihalo steroids are readily prepared according to the processes disclosed in U.S. Patent Application Serial No. 711,042 filed August 2, 1976 by formylating an appropriate 16α,17α-isopropylidenedioxy-pregna-1,4,9(11)-triene-3,20-dione with ethyl formate in a hindered base to form a hydroxymethylene group at 21; halogenating at 21 with, e.g., lithium chloride, cupric bromide, or perchloryl fluoride to form a 21,21-dihalo-21-formyl; and deformylating with sodium hydroxide to form the 21,21-dihalo steroid.

To prepare the 21,21-dihydroxy and 21,21-dialkoxy steroids useful as starting materials, the process set forth in U.S. 4,011,315 is particularly useful and that patent is incorporated herein by reference. In this process, an appropriate 21-hydroxy steroid is contacted with air in the presence of a copper (II) catalyst such as cupric acetate to yield the 21,21-dihydroxy steroid (i.e. a 21-aldehyde hydrate). The rection is preferably conducted in methanol at a temperature of 50° to 30°C for a period of 30 minutes to 60 hours.

The 21-aldehyde hydrate can then be isolated according to methods known in the art or may then be heated under vacuum at a temperature of 100°C for a period of 30 minutes to 3 hours to yield the 21-aldehyde which is reacted with a lower alkanol containing 1 to 8 carbon atoms, preferably methanol, at a temperature of 20° to 60°C for a period of 15 minutes to 1 hour to yield a 21-aldehyde hemiacetal.

Reaction of the 21-aldehyde hemiacetal with a suitable halogenating agent such as methane sulfonyl chloride, thionyl chloride, or thionyl bromide in the presence of an organic base such as triethylamine, pyridine and the like yields the 21-halo-21-alkyl ether. The reaction is preferably conducted in a chlorinated organic solvent such as methylene chloride, chloroform, 1,1-dichloroethane and the like for a period of 1 to 16 hours and a temperature of -20° to +10°C. The thus obtained 21-halo-21-alkyl ether is then treated with an alkali metal alkoxide, preferably a sodium alkoxide such as sodium methoxide. The reaction is conducted in a solvent medium usually containing the alkanol corresponding to the alkoxide utilized although a solvent inert to the other reactants may be employed.

Reaction temperatures are preferably maintained at 20° to 60°C for a period of 2 to 6 hours.

By following the procedures set forth above for preparing the starting material for the compounds of this invention, steroids of a relatively simple structure can be converted to other structures as desired. Thus, exemplary known compounds which can be employed to prepare starting materials for compounds of this invention according to procedures discussed above include progesterone, corticosterone, hydrocortisone, prednisolone, betamethasone, dexamethasone, triamcinolone, paramethasone, fluocinolone, triamcinolone acetonide, fluocinolone acetonide, and the like.

Further specific embodiments of this invention are found in the following examples which are given by way of illustration only and are not to be interpreted as limiting the scope of the claims appended hereto.

Example 1

This example sets forth a process for preparing 4,6α,9α-trifluoro-11β-hydroxy-16α,17α-isopropylidene-dioxypregna-1,4-diene-3,20-diones (which are substituted at the 21 position) according to the following reaction sequence:

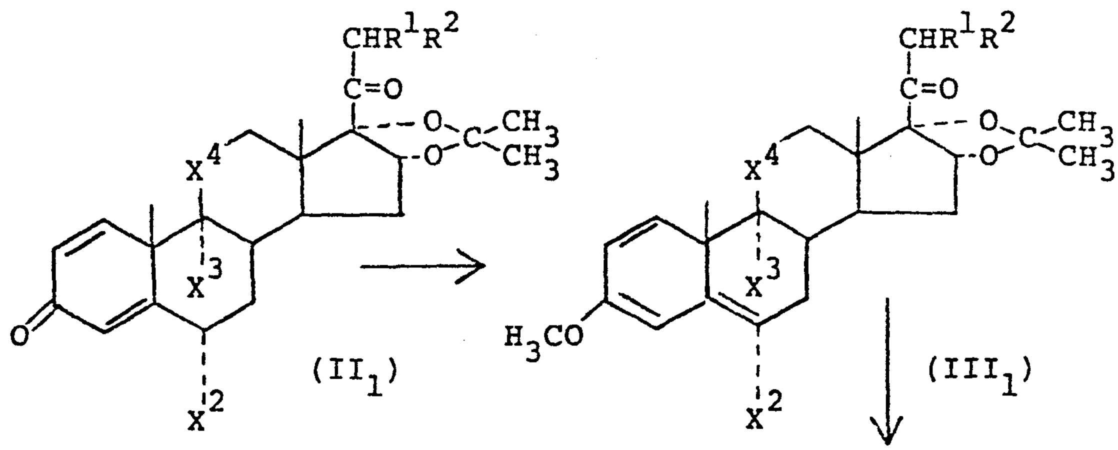

($II_1$) ($III_1$)

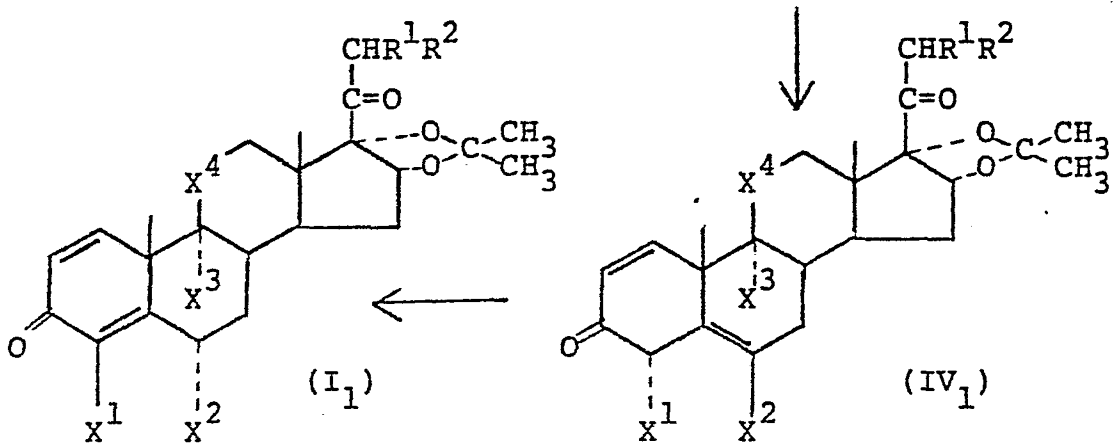

$(I_1)$ $(IV_1)$

A. Preparation of 4,6α,9α-trifluoro-11β,21-dihydroxy-16α,17α-isopropylidenedioxypregna-1,4-diene-3,20-dione.

Ten grams (g) fluocinolone acetonide (6α,9α-difluoro-11β,21-dihydroxy-16α,17α-isopropylidenedioxy-pregna-1,4-diene-3,20-dione) is stirred into 5 milliliters (ml) of acetic anhydride, 5 ml triethylamine and 1 g 4-N,N-dimethylamino pyridine at room temperature for 5 hours to form the corresponding 11β,21-diacetate ($X^2$ and $X^3$ are F, $X^4$ is $=C<^{O(O)CCH_3}_{H}$, $R^2$ is acetoxy and $R^1$ is H) which is then recovered by precipitation in water and filtration.

To a solution of 620 ml trimethyl orthoformate, 206 ml anhydrous methanol, and 4.1 ml fuming sulfuric acid, 42 g of the 11β,21-diacetate of fluocinolone acetonide prepared according to the preceeding paragraph is added. The resulting mixture is stirred at 50-55°C for 30 minutes, at which time thin layer chromatography (TLC) using an eluant of 35% ethyl acetate and 65% hexane shows that the reaction is complete. Twenty-five ml of triethyl amine are added to neutralize the acid and the solvents are removed using a rotary evaporator at reduced pressure. The residue is dissolved in 500 ml of acetone, about 25 ml water is added and the acetone is removed under reduced pressure to give crystalline

precipitate which is collected by filtration and air dried overnight to give 40 g. of compound (III ), above, wherein $R^1$ is hydrogen, $R^2$ is acetoxy, $R^4$ is $=C\genfrac{}{}{0pt}{}{O(O)CCH_3}{H}$ and $X^2$ and $X^3$ are F.

Thirty g of the resulting product is added to 300 ml of a solvent consisting of 90% by weight (%w) acetone and 10%w water and molar equivalent of perchloryl fluoride ($ClO_3F$) is added at ambient temperature over about 30 minutes. TLC of the reaction mixture using a mixture of 35% ethyl acetate and 65% hexane shows the reaction to be complete upon completion of the $ClO_3F$ addition. Water is slowly added to the reaction mixture until a total volume of 2 l is obtained. A crystalline precipitate forms which is collected by filtration, dissolved in 300 ml methylene chloride ($CH_2Cl_2$) and the resulting solution is dried over anhydrous sodium sulfate. The product represented by ($IV_1$) wherein $R^1$ is hydrogen; $R^2$ is acetoxy; $X^1$, $X^2$ and $X^3$ are F; and $X^4$ is $=C\genfrac{}{}{0pt}{}{O(O)CCH_3}{H}$ is purified by chromatography on 300 g column of silica gel, eluting with a mixture of 75% $CH_2Cl_2$ and 25% hexane. The homogeneous fractions are combined, concentrated to dryness, and the residue is crystallized from $CH_2Cl_2/CH_3OH$ to give 15 g of a compound represented by formula ($IV_1$) wherein $R^1$ is hydrogen and R and $R^2$ are each acetoxy.

One g of the resulting product is stirred with 20 ml methanol and 20 ml $CH_2Cl_2$ containing 200 milligrams (mg) of anhydrous potassium carbonate under nitrogen at atmospheric pressure and ambient temperature for one hour, at which time TLC shows the reaction is complete. The reaction mixture is diluted with 20 ml methanol and 2 ml glacial acetic acid and concentrated under reduced pressure to a small volume. The crystalline precipitate which forms is collected by filtration and washed with methanol and water to give 800 mg of the final product,

namely 4,6α,9α-trifluoro-11β,21-dihydroxy-16α,17α-isopropylidenedioxypregna-1,4-diene-3,20-dione, melting point (mp) 270-271.5°C, $_D$[α] 108° ($CHCl_3$).

B. Ten g of the final product from step A are heated with 10 ml mesyl chloride in 100 ml pyridine at 0°C for 1 hour and the resulting mixture is poured into water to form a precipitate which is filtered and air dried to give the 21-mesylate corresponding to formula ($I_1$), wherein $R^1$ is H, $R^2$ is a mesyl ester and R is hydroxy. Ten g of this material is reacted with lithium chloride in 100 ml sulfolane at 100°C for 8 hours. Water is added to form a precipitate which is collected by filtration, air dried and recrystallized from $CH_2Cl_3$-MEOH to give 4,6α,9α-trifluoro-11β-hydroxy-16α,17α-isopropylidenedioxy-21-chloropregna-1,4-diene-3,20-dione, m.p. 285.5-289.0°C, $[α]_D$(Pyridine).

C. By following the procedure set forth in part A of this example but substituting the appropriate starting material for fluocinolone acetonide the following compounds are prepared:

4,6α,9α,21-tetrafluoro-11β-hydroxy-16α,17α-isopropylidenedioxypregna-1,4-diene-3,20-dione, m.p. 280-281°C;

4,6α,9α,21,21-pentafluoro-11β-hydroxy-16α,17α-isopropylidenedioxypregna-1,4-diene-3,20-dione;

4,6α,9α-trifluoro-11β-hydroxy-16α,17α-isopropylidenedioxy-21,21-dichloropregna-1,4-diene-3,20-dione;

4,6α,9α-trifluoro-11β-hydroxy-16α,17α-isopropylidenedioxy-21,21-dimethoxypregna-1,4-diene-3,20-dione, m.p. 268-270°C, $[α]_D$ 30° ($CHCL_3$);

4,6α,9α-trifluoro-11β,21-dihydroxy-16α,17α-isopropylidenedioxypregna-1,4-diene-3,20-dione;

4,6α,9α-trifluoro-11β-hydroxy-16α,17α-isopropylidenedioxy-21-acetoxypregna-1,4-diene-3,20-dione;

4,6α,9α-trifluoro-11β-hydroxy-16α,17α-isopropylidenedioxy-21-propionyloxypregna-1,4-diene-3,20-dione and

the like.

Example 2

This example sets forth a process for preparing 4,6α-difluoro-9α,11β-dichloro-16α,17α-isopropylidenedioxypregna-1,4-diene-3,20-diones (which are substituted at the 21 position) according to the reaction of Example wherein $X^1$ and $X^2$ are both fluoro; $X^3$ and $X^4$ are both chloro; $R^1$ is hydrogen, fluoro, chloro, hydroxy or methoxy; and $R^2$ is fluoro, chloro, hydroxy or alkanoyloxy of 2 through 6 carbon atoms when $R^1$ is hydrogen or $R^2$ is the same as $R^1$ when $R^1$ is fluoro, chloro, hydroxy or methoxy.

A. 4,6α,21-Trifluoro-9α,11β-dichloro-16α,17α-isopropylidenedioxypregna-1,4-diene,2,30-dione.

To a solution of 620 ml trimethyl orthoformate, 206 ml anhydrous methanol, and 4.1 ml fuming sulfuric acid, is added 40 g of 6α,21-difluoro-9α,11β-dichloro-16α,17α-isopropylidenedioxypregna-1,4-diene-3,20-dione. The resulting mixture is stirred at 50-55°C for 30 minutes, at which time thin layer chromatography (TLC) using an eluant of 35% ethyl acetate and 65% hexane shows that the reaction is complete. Twenty-five ml of triethylamine are added to neutralize the acid and the solvents are removed using a rotary evaporator at reduced pressure. The residue is dissolved in 500 ml of acetone, about 25 ml water is added and the acetone is removed under reduced pressure to give a crystalline precipitate which is collected by filtration and air dried over night to give 39 g of compound ($III_2$), above, wherein $R^1$ is hydrogen, $R^2$ is fluoro, $X^2$ is fluoro, $X^3$ is chloro and $X^4$ is $=C{<}^{Cl}_{H}$.

Thirty g of the resulting product are added to 300 ml of a solvent consisting of 90% acetone and 10% water

and a molar equivalent of $ClO_3F$ are added at ambient temperture over about 30 minutes. TLC of the reaction mixture using an eluant of 35%w ethylacetate and 65%w hexane shows the reaction to be complete upon completion of the $ClO_3F$ addition. Water is slowly added to the reaction mixture until a total volume of two liters is obtained. The crystalline precipitate which forms is collected by filtration, dissolved in $CH_2Cl_2$ and the resulting solution is dried over anhydrous sodium sulfate. The product represented by Formula ($IV_2$) wherein $R^1$, $R^2$, $X^2$, $X^3$ and $X^4$ are as defined in this example and $X^1$ is fluoro is purified by chromatography on a 300 g silica gel column eluting with a mixture of 75% $CH_2Cl_2$ and 25% hexane. The homogeneous fractions are combined, concentrated to dryness, and the residue crystallized from $CH_2Cl_2/CH_3OH$ to give 15 g of a compound represented by Formula ($IV_1$) wherein $R^1$, $R^2$, $X^1$, $X^2$, $X^3$ and $X^4$ are defined in this example.

One g of the resulting product is stirred with 20 ml methanol and 20 ml $CH_2Cl_2$ containing 200 mg of anhydrous potassium carbonate under nitrogen at atmospheric pressure and ambient temperature for one hour, at which time TLC show the reaction is complete. The reaction mixture is diluted with 20 ml methanol and 2 ml glacial acetic acid and concentrated under reduced pressure to a small volume. The crystalline precipitate which forms is collected by filtration and washed with methanol and water to give .0 g of 4,6α,21-trifluoro-9α,11β-dichloro-16α,17α-isopropylidenedioxypregna-1,4-diene-3,20-dione, m.p. $[\alpha]_D$ 149° ($CHCl_3$).

B. By following in principle the procedure set forth in part A of this example and using an appropriate starting material as set forth in the "Preparation of Starting Compounds" part of the specification, the following compounds are prepared:

4,6α,21,21-tetrafluoro-9α,11β-dichloro-16α,17α-isopropylidenedioxypregna-1,4-diene-3,20-dione;

4,6α,21-trifluoro-9α,11β,21-trichloro-16α,17α-isopropylidenedioxypregna-1,4-diene-3,20-dione;

4,6α-difluoro-9α,11β,21,21-tetrachloro-16α,17α-isopropylidenedioxypregna-1,4-diene-3,20-dione;

4,6α-difluoro-9α,11β,21-trichloro-16α,17α-isopropylidenedioxypregna-1,4-diene-3,20-dione, mp 254.5-256.5°C, $[\alpha]_D$+164 ($CHCl_3$);

4,6α-difluoro-9α,11β-dichloro-16α,17α-isopropylidenedioxy-21,21-dimethoxypregna-1,4-diene-3,20-dione;

4,6α-difluoro-9α,11β-dichloro-16α,17α-isopropylidenedioxy-21-hydroxypregna-1,4-diene-3,20-dione;

4,6α-difluoro-9α,11β-dichloro-16α,17α-isopropylidenedioxy-21-valeryloxypregna-1,4-diene-3,20-dione;

4,6α-difluoro-9α,11β-dichloro-16α,17α-isopropylidenedioxy-21-acetoxy-1,4-dione-3,20-dione; and the like.

## Example 3

This example sets forth a process for preparing 4-chloro-6α,9α-difluoro-11β-hydroxy-16α,17α-isopropylidenedioxypregna-1,4-diene-3,20-diones (which are substituted at the 21 position similarly to Example 2) according to the reaction sequence of Example 1 wherein $X^1$ is chloro, $X^2$ and $X^3$ are both fluoro and $X^4$ is $-C{\lt}^{OH}_{H}$.

A. By following in principle the procedure of Example 1, Part A but substituting 21-chloro-6α,9α-difluoro-11β-hydroxy-16α,17α-isopropylidenedioxypregna-1,4-diene-3,20-dione for fluocinolone acetonide and dichlorohydantoin for $ClO_3F$, 4,21-dichloro-6α,9α-difluoro-11β-hydroxy-16α,17α-isopropylidenedioxypregna-1,4-diene-3,20-dione, mp 286-289°C, $[\alpha]_D$+161° (pyridine), is obtained.

B. By following in principle the procedure of Part A of this example but substituting an appropriate

starting material as set forth in the "Preparation of Starting Compounds" section for fluocinolone acetonide the following compounds are obtained:

4-chloro-6α,9α,21-trifluoro-11β-hydroxy-16α,17α-isopropylidenedioxypregna-1,4-diene-3,20-dione;

4-chloro-6α,9α,21,21-tetrafluoro-11β-hydroxy-16α,17α-isopropylidenedioxypregna-1,4-diene-3,20-dione;

4,21,21-trichloro-6α,9α-difluoro-11β-hydroxy-16α,17α-isopropylidenedioxypregna-1,4-diene-3,20-dione;

4-chloro-6α,9α-difluoro-11β-hydroxy-16α,17α-isopropylidenedioxy-21,21-dimethoxypregna-1,4-diene-3,20-dione;

4-chloro-6α,9α-difluoro-11β,21-dihydroxy-16α,17α-isopropylidenedioxypregna-1,4-diene-3,20-dione;

4-chloro-6α,9α-difluoro-11β-hydroxy-16α,17α-isopropylidenedioxy-21-acetoxypregna-1,4-diene-3,20-dione;

4-chloro-6α,9α-difluoro-11β-hydroxy-16α,17α-isopropylidenedioxy-21-propionyloxypregna-1,4-diene-3,20-dione; and the like.

## Example 4

This example sets forth a process for preparing 4,9α,11β-trichloro-6α-fluoro-16α,17α-isopropylidenedioxypregna-1,4-diene-3,20-dione (which are substituted at the 21 position similarly to Example 2) according to the reaction sequence of Example 1 wherein $X^1$ and $X^3$ are chloro, $X^2$ is fluoro, and $X^4$ is $=C\begin{smallmatrix}OH \\ H\end{smallmatrix}$.

A. By following in principle the procedure of Example 2, Part A but substituting 9α,11α,21-trichloro-6α-fluoro-16α,17α-isopropylidenedioxypregna-1,4-diene-3,20-dione for 6α,21-difluoro-9α,11β-dichloro-16α,17α-isopropylidenedioxypregna-1,4-diene-3,20-dione and dichlorohydantoin for $ClO_3F$, 4,9α,11β-21-tetrachloro-6α-fluoro-16α,17α-isopropylidenedioxypregna-1,4-diene-3,20-dione, mp 256-259°C, $[\alpha]_D$+183°($CHCl_3$), is obtained.

B. By following in principle the procedure of Part A of this example, but substituting an appropriate starting material as prepared in the "Preparations of Starting Compounds" section, the following compounds are obtained:

4,9α,11β-trichloro-6α,21-difluoro-16α,17α-isopropylidenedioxypregna-1,4-diene-3,20-dione, mp 257.5-258°C, $[\alpha]_D$+102° ($CHCl_3$);

4,9α,11β-trichloro-6α,21,21-trifluoro-16α,17α-isopropylidenedioxypregna-1,4-diene-3,20-dione;

4,9α,11β,21,21-pentachloro-6α-fluoro-16α,17α-isopropylidenedioxypregna-1,4-diene-3,20-dione;

4,9α,11β,21-tetrachloro-6α-fluoro-16α,17α-isopropylidenedioxypregna-1,4-diene-3,20-dione;

4,9α,11β-trichloro-6α-fluoro-16α,17α-isopropylidenedioxy-21,21-dimethoxypregna-1,4-diene-3,20-dione;

4,9α,11β-trichloro-6α-fluoro-16α,17α-isopropylidenedioxy-21-hydroxypregna-1,4-diene-3,20-dione;

4,9α,11β-trichloro-6α-fluoro-16α,17α-isopropylidenedioxy-21-hexanoylhydroxypregna-1,4-diene-3,20-dione;

4,9α,11β-trichloro-6α-fluoro-16α,17α-isopropylidenedioxy-21-acetoxypregna-1,4-diene-3,20-dione, and the like

## Example 5

This example sets forth a process for preparing 4,9α-difluoro-6-chloro-11β-hydroxy-16α,17α-isopropylidenedioxypregna-1,4-diene-3,20-diones (which are substituted at the 21 position similarly to Example 2) according to the reaction sequence of Example 1 wherein $X^1$ and $X^3$ are fluoro, $X^2$ is chloro and $X^4$ is $=C{<}^{OH}_{H}$.

A. 4,9α-Difluoro-6α-chloro-11β,21-dihydroxy-16α,17α-isopropylidenedioxypregna-1,4-diene-3,20-dione is prepared by following in principle the procedure set forth in Example 1, Part A, but substituting 6α-chloro-9α-fluoro-11β,21-dihydroxy-16α,17α-isopropylidenedioxy-

pregna-1,4-diene-3,20-dione for fluocinolone acetonide.

B. By substituting other appropriate starting materials in the process of Part A of this example other compounds of this invention are prepared such as

4,9α-difluoro-6α,21-dichloro-11β-hydroxy-16α,17α-isopropylidenedioxypregna-1,4-diene-3,20-dione;

4,9α-difluoro-6α-chloro-11β-hydroxy-16α,17α-isopropylidenedioxy-21,21-dimethoxypregna-1,4-diene-3,20-dione; and the like.

<u>Example 6</u>

This example sets forth a process for preparing 4-fluoro-6α,9α,11β-trichloro-11β-hydroxy-16α,17α-isopropylidenedioxypregna-1,4-diene-3,20-diones (which are substituted at the 21 position similarly to Example 2) according to the reaction sequence of Example 1 wherein $X^1$ is fluoro, $X^2$ and $X^3$ are both chloro and $X^4$ is $=C<^{Cl}_{H}$.

A. 4,21-Difluoro-6α,9α,11β-trichloro-16α,17α-isopropylidenedioxypregna-1,4-diene-3,20-dione is prepared by following in principle the procedure of Example 2, Part A but substituting 6α,9α,11β-trichloro-16α,17α-isopropylidenedioxy-21-fluoropregna-1,4-diene-3,20-dione for 6α,21-difluoro-9α,11β-dichloro-16α,17α-isopropylidenedioxypregna-1,4-diene-3,20-dione as the starting material.

B. By substituting other appropriate starting materials in the process of Part A of this example, other compounds of this invention are prepared such as

4-fluoro-6α,9α,11β-trichloro-16α,17α-isopropylidenedioxy-21-acetoxypregna-1,4-diene-3,20-dione;

4-fluoro-6α,9α,11β,21-tetrachloro-16α,17α-isopropylidenedioxypregna-1,4-dione; and the like.

Example 7

By following in principle the process set forth in Example 1, Part A but deleting the acetylation step and substituting an appropriate 21-substituted 6α,9α-dichloro-11β-hydroxy-16α,17α-isopropylidenedioxypregna-1,4-diene-3,20-dione for fluocinolone acetonide, 4-fluoro-6α,9α-dichloro-11β-hydroxy-16α,17α-isopropylidene dioxypregna-1,4-diene-3,20-diones are prepared according to the reaction sequence of Example 1 wherein $R^1$ is hydrogen, fluoro, chloro, bromo, hydroxy or methoxy and $R^2$ is fluoro, chloro, bromo, hydroxy or alkanoyloxy of 2-6 carbons when $R^1$ is hydrogen, or $R^2$ is the same as $R^1$ when $R^1$ is fluoro, chloro or methoxy.

In this process, however, the conversion from the 4α-fluoro-6-chloropregna-1,5-diene to the corresponding 4-fluoro-6α-chloropregna-1,4-diene takes place under acid conditions instead of basic conditions set forth in Example 1. Thus, ten g of the compound represented by $(IV_1)$ (wherein $R^1$ is hydrogen, $R^2$ is hydroxy, $X^1$ is fluoro, $X^2$ and $X^3$ are chloro, and $X^4$ is $=C\begin{smallmatrix}OH\\H\end{smallmatrix}$) is placed in 100 ml $CH_3OH$ along with one ml of concentrated HCl and the resulting mixture is refluxed for two hours. The reaction mixture is thereafter poured into about 500 ml water and the resulting precipitate is extracted with $CH_2Cl_2$. The $CH_2Cl_2$ solution is washed with three equal volumes of water, dried over anhydrous sodium sulfate, concentrated to dryness and the resulting precipitate is purified using column chromatography to give 4-fluoro-6α,9α-dichloro-11β-hydroxy-16α,17α-isopropylidenedioxy-21-hydroxypregna-1,4-diene-3,20-dione.

Example 8

By following in principle the process set forth in Example 7, but substituting an appropriate, 21-substi-

tuted 6α,9α-dichloro-11β-hydroxy-16α,17α-isopropylidene-dioxypregna-1,4-diene-3,20-dione for 21-chloro-6α,9α-difluoro-11β-hydroxy-16α,17α-isopropylidenedioxypregna-1,4-diene-3,20-dione and dichlorohydantoin for $ClO_3F$, the desired 4,6α9α-trichloro-11β-hydroxy-16α,17α-isopropylidenedioxypregna-1,4-diene-3,20-diones are prepared according to the reaction sequence of Example 1 wherein $R^1$ is hydrogen, fluoro, chloro, bromo, hydroxy or methoxy and $R^2$ is fluoro, chloro, bromo, hydroxy or alkanoyloxy of 2-6 carbons when $R^1$ is hydrogen or is the same as $R^1$ when $R^1$ is fluoro, chloro or methoxy.

Example 9

This example sets forth a process for preparing 21-substituted 4,6α-difluoro-11β-hydroxy-16α,17α-isopropylidenedioxypregna-4-ene-3,20-diones according to the following reaction sequence:

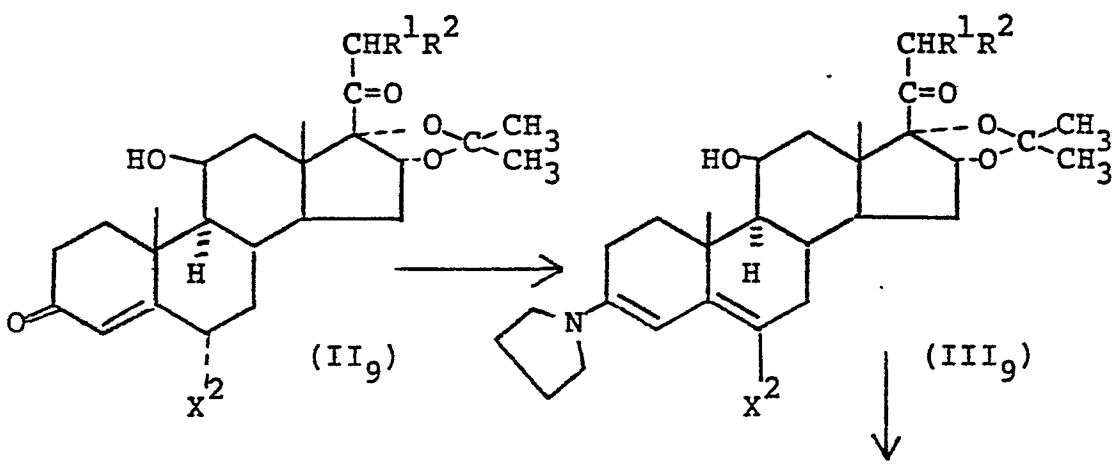

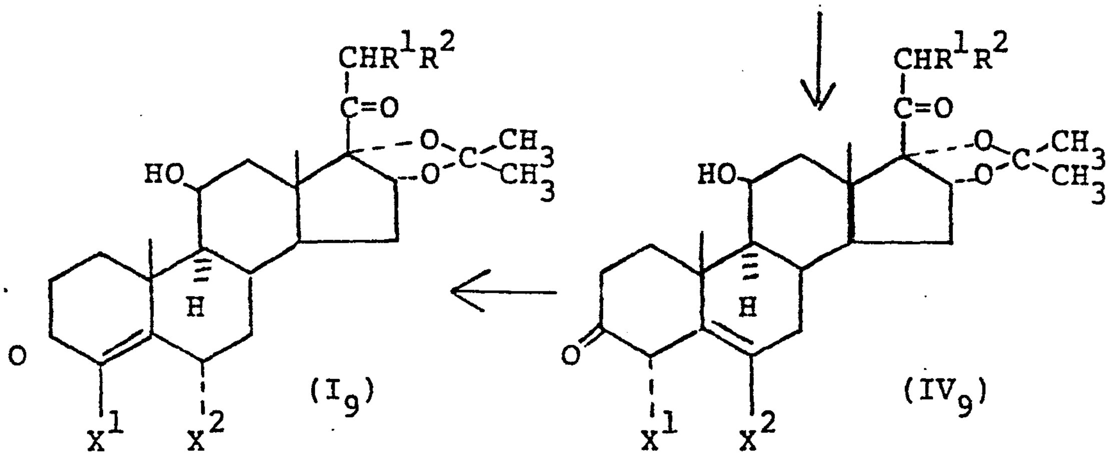

$(I_9)$ $(IV_9)$

wherein $R^1$ and $R^2$ are as defined in Example 8 and $X^1$ and $X^2$ are both fluoro.

Ten g of a compound of formula $(II_9)$ are mixed with 100 ml benzene and refluxed in the presence of a slight molar excess of pyrrolidine until TLC analysis indicates that the reaction is complete. Excess benzene and pyrrolidine are eliminated under reduced pressure to leave a residue of an enamine represented by formula $(III_9)$ which is purified by crystallization from a suitable organic solvent or by chromatography using alumina.

One g of compound of formula $(III_9)$ is dissolved in 10 ml pyridine and the resulting solution is cooled to 0°C, then perchloryl fluoride, is slowly bubbled into the reaction mixture, until TLC analysis indicates the reaction is complete. The mixture is then concentrated under reduced pressure to give a crude residue of a compound represented by formula $(IV_9)$ which is purified by crystallization from a suitable solvent or by chromatography on silica gel.

Five hundred (500) mg of a compound represented by formula $(IV_9)$ is dissolved in MeOH or a mixture of MeOH and dichloromethane, under a nitrogen atmosphere and a catalytic amount (20% of an equivalent) of anhydrous potassium carbonate is added. The reaction

mixture is stirred at room temperature until TLC analysis indicates the reaction is complete. Upon completion of the reaction, glacial acetic acid is added to destroy excess potassium carbonate, the mixture is concentrated, the mixture is concentrated under reduced pressure to a small volume and water is added thereto to give a crystalline precipitate of a compound represented by formula ($I_9$). If a C-21 acetate is present in the starting material, it will be hydrolyzed to the corresponding C-21 alcohol.

By substituting dichlorohydantoin for $ClO_3F$ in this example, the corresponding 4-chloro steroid is obtained.

Example 10

By following in principle the procedure and reaction sequence of Claim 9, 21-substituted 4,6α-dichloro-11β-hydroxy-16,17α-isopropylidenedioxypregn-4-ene-3,20-diones are prepred wherein $X^1$ and $X^2$ are both chloro.

In this reaction sequence, however, to convert a compound represented by formula ($III_9$) to one of formula (IV ) dichlorohydantoin is substituted for perchloryl fluoride. Thus, a compound of formula ($III_9$) is dissolved in a mixture of 90% acetone and 10% water and reacted with 1.1 equivalents of dichlorohydantoin at room temperature for about 30 minutes. The resulting mixture is diluted with water and the acetone is eliminated by distillation under reduced pressure to give a crystalline precipitate which is extracted with $CH_2Cl_2$ and purified as discussed in Example 9.

Example 11

This example sets forth a process for preparing 4,6α,9α-trifluoro-11α,17α,21-trihydroxy-16α-methylpregna-1,4-diene-3,20-diones according to the following

reaction sequence:

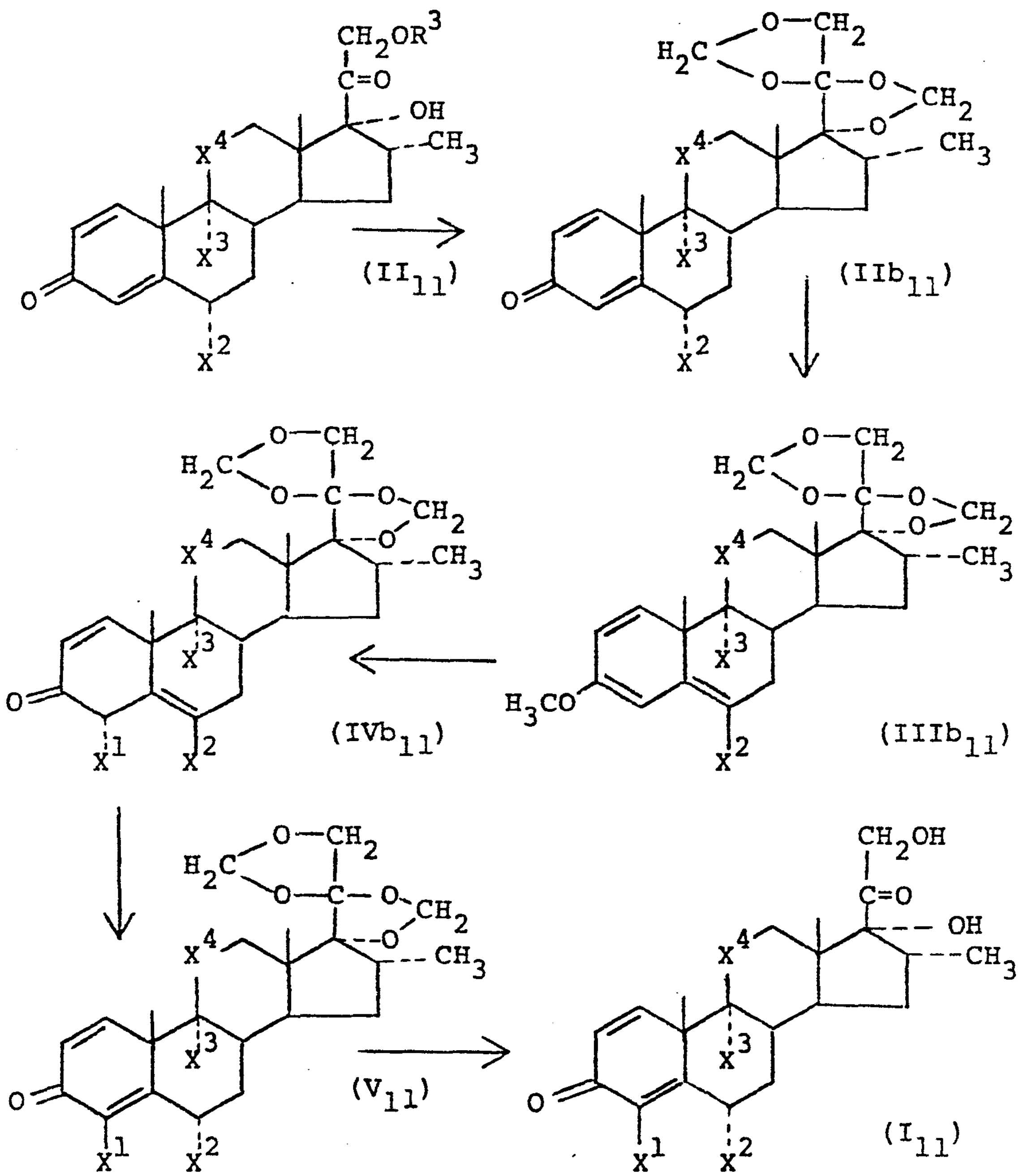

$(II_{11})$ $(IIb_{11})$ $(IIIb_{11})$ $(IVb_{11})$ $(V_{11})$ $(I_{11})$

A. Preparation of 4,6α,9α-trifluoro-11β,17α,21-trihydroxy-16α-methylpregna-1,4-diene-3,20-dione.

Flumethasone (6α,9α-difluoro-11β,17α,21-trihydroxy-16α-methylpregna-1,4-diene-3,20-dione: 8 grams) is stirred into 200 milliliters (ml) of chloroform, and 50

g of paraformaldehyde and 120 ml of conc. HCl in 120 ml of water. (The paraformaldehyde solution is warmed to achieve dissolution.) The mixture is stirred for 48 hours at ambient temperature and the two layers are separated. The aqueous layer is extracted with chloroform, and the combined organic layer and chloroform extracts are washed with water to neutrality, dried over sodium sulfate and evaporated to dryness to yield the 17,20:20,21-bismethylenedioxy (BMD) derivative of flumethasone ($IIb_{11}$) which is recrystallized from methanol:ether.

To a solution of 620 ml trimethyl orthoformate, 206 ml anhydrous methanol, and 4.1 ml fuming sulfuric acid, is added 41 g of the BMD derivative of flumethasone prepared according to the preceeding paragraph. The resulting mixture is warmed to 40-50° for 30 minutes, at which time thin layer chromatography (TLC) using an eluant of 35% ethyl acetate and 65% hexane shows that the reaction is complete. Twenty-five ml of triethyl amine are added to neutralize the acid and the solvents are removed using a rotary evaporator at reduced pressure. The residue is dissolved in 500 ml of acetone, about 240 ml water is added and the acetone is removed under reduced pressure to give crystalline precipitate which is collected by filtration and air dried overnight to give 50 g of a compound represented by formula ($IIIb_{11}$), (where $X^2$ and $X^3$ are fluoro and $X^4$ is $=C{<}^{OH}_{H}$) which is recrystallized from methanol-water.

The resulting product (10 g) is added to 300 ml of a solvent consisting of 90% acetone and 10% water and a slow stream of perchloryl fluoride ($ClO_3F$) is added at ambient temperature over about 30 minutes. TLC of the reaction mixture using a mixture of 35% ethyl acetate and 65% hexane shows the reaction to be complete upon completion of the $ClO_3F$ addition. Water is slowly added

to the reaction mixture until a total volume of 2 l is obtained. The mixture is concentrated under reduced pressure to give a crystalline precipitate which is collected by filtration, dissolved in methylene chloride ($CH_2Cl_2$) and the resulting solution is dried over anhydrous sodium sulfate. The solution containing the product represented by ($IVb_{11}$) (where $X^1$, $X^2$, $X^3$ and $X^4$ are as defined), is purified by chromatography in silica gel. The solution is concentrated to dryness to give the product represented by formula ($IVb_{11}$). Five g. of this product are added to a mixture of 50 g of urea/-liquid hydrofluoric acid and 10 ml of water, the mixture is stirred at room temperature for 2 hours, at which time TLC in 5% methanol-95% dichloromethane shows the reaction to be complete. The reaction mixture is then diluted with one l of water, and the crystalline precipitate is collected by filtration and dried to give about 3.5 g of the BMD product represented by formula ($V_{11}$) (where $X^1$-$X^4$ are as defined). One g of the BMD compound is mixed with 20 ml of 60% formic acid and heated on a steam bath for 1 hour. The mixture is cooled, diluted with water, and the resulting precipitate is filtered, water washed, dried and recrystallized from acetone:hexane to yield 4,6α,9α-trifluoro-11β,17,21-trihydroxy-16α-methylpregna-1,4-diene-3,20-dione.

B. By following in principle the procedure set forth in part A (using the BMD intermediate only if there is a 17,21 dihydroxy starting material) of this example but substituting the appropriate starting material for flumethasone the following compounds are prepared.

4,6α,9α,21-tetrafluoro,11β,17α-dihydroxy-16α-methyl-pregna-1,4-diene-3,20-dione;

4,6α,9α-trifluoro-11β,17α-dihydroxy-16α-methyl-21-chloropregna-1,4-diene-3,20-dione;

4,6α,9α-trifluoro-11β-hydroxy-16α-methyl-17α-propionyloxy-21-chloropregna-1,4-diene-3,20-dione;

4,6α,9α-trifluoro-11β,17α-dihydroxy-16α-methyl-21-chloropregna-1,4-diene-3,20-dione;

4,6α,9α-trifluoro-11β,17α-dihydroxy-16α-methyl-21,21-dimethoxypregna-1,4-diene-3,20-dione;

4,6α,9α--trifluoro-11β,17α-dihydroxy-16α-methyl-21-hydroxypregna-1,4-diene-3,20-dione;

4,6α,9α-trifluoro-11β,17α-dihydroxy-16α-methyl-21-acetoxypregna-1,4-diene-3,20-dione;

4,6α,9α-trifluoro-11β,17α-dihydroxy-16α-methyl-21-propionyloxypregna-1,4-diene-3,20-dione; and the like.

## Example 12

The example sets forth a process for preparing 4,6α-difluoro-9α,11β-dichloro-16α-methyl-17α-hydroxy-pregna-1,4-diene-3,20-diones (which are substituted at the 21 position similarly to Example 11) according to the following reaction sequence wherein $X^1$ and $X^2$ are fluoro, $X^3$ is chloro and $X^4$ is $=C\begin{smallmatrix}OH\\H\end{smallmatrix}$ :

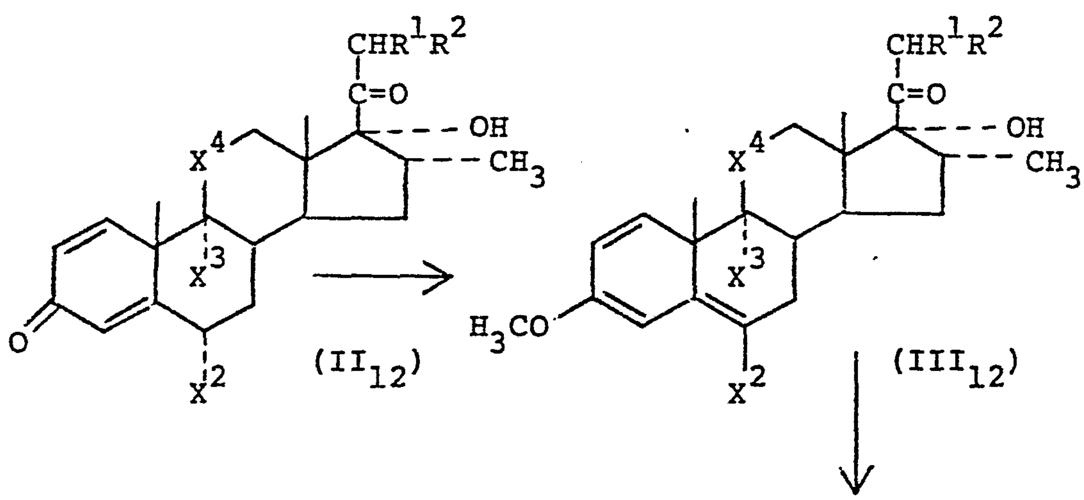

($II_{12}$) ($III_{12}$)

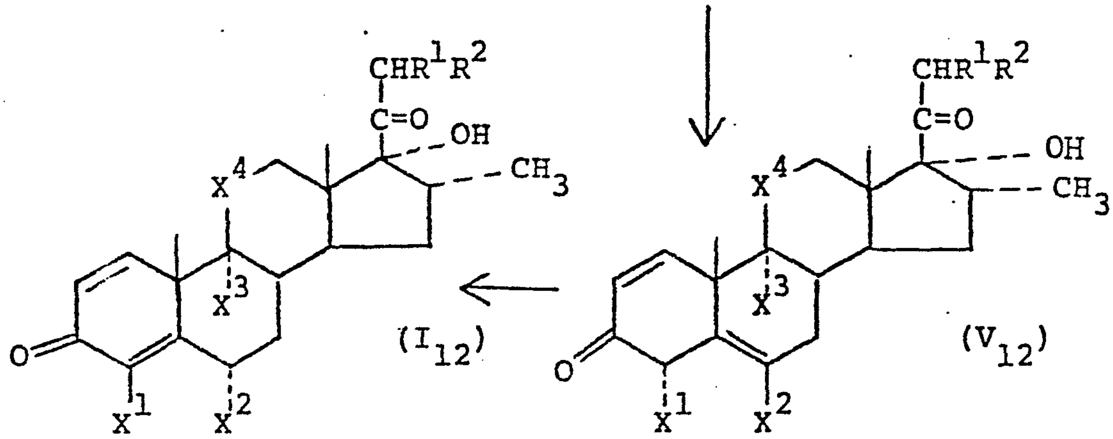

$(I_{12})$ $(V_{12})$

A. 4,6α,21-trifluoro-9α,11β-dichloro-16α-methyl-17α-hydroxypregna-1,4-diene-3,20-dione.

To a solution of 620 ml trimethyl orthoformate, 206 ml anhydrous methanol, and 4.1 ml fuming sulfuric acid, 41 g of 6α,21-difluoro-9α,11β-dichloro-16α-methyl-17α-hydroxypregna-1,4-diene-3,20-dione is added. The resulting mixture is heated to 40-55° for 30 minutes, at which time TLC using an eluant of 35% ethyl acetate and 65% hexane shows that the reaction is complete. Twenty-five ml of triethylamine are added to neutralize the acid and the solvents are removed using a rotary evaporator at reduced pressure. The residue is dissolved in 500 ml of acetone, about 25 ml water is added and the acetone is removed under reduced pressure to give a crystalline precipitate which is collected by filtration and air dried overnight. The solid is dissolved in 400 ml of 75% methylene chloride/25% hexane and adsorbed on a column of 400 g silica gel. Continued elution with 80% methylene chloride/20% hexane, pure methylene chloride, then methylene chloride containing varying concentrations of ethyl acetate to give a series of portions which were combined then evaporated to leave about 12 g of a compound, represented by formula $(III_{12})$ wherein $R^1$ is hydrogen and $R^2$ is fluoro.

The resulting product (10 g) is added to 300 ml of a mixture of 80% tetrahydrofuran and 20% water and a

slow current of perchloryl fluoride ($ClO_3F$) is bubbled through the reaction mixture at ambient temperature over about 30 minutes. TLC of the reaction mixture using a mixture of 35% ethylacetate and 65% hexane shows the reaction to be complete upon completion of the $ClO_3F$ addition. Water is slowly added to the reaction mixture until a total volume of 600 ml is obtained. The solvent acetone is eliminated under reduced pressure and the crystalline material is collected by filtration and purified by chromatography over a silica gel using a solvent mixture consisting of $CH_2Cl_2$ and hexane.

The resulting product (1 g) is stirred with 20 ml methanol and 20 ml $CH_2Cl_2$ containing 200 milligrams (ml) of anhydrous potassium carbonate in an inert atmosphere (nitrogen) at atmospheric pressure and ambient temperature for one hour, at which time TLC shows the reaction is complete. The reaction mixture is diluted with 20 ml methanol and 2 ml glacial acetic acid and concentrated under reduced pressure to a small volume. The crystalline precipitate which forms is collected by filtration and washed with methanol and water to give 0.5 g of the final product, namely 4,6α,21-trifluoro-9α,11β-dichloro-16α-methyl-17α-hydroxypregna-1,4-diene-3,20-dione.

B. By following in principle the procedure set forth in part A of this example and part A of Example 12, if appropriate, and using an appropriate starting material as prepared according to principles as set forth in the "Preparation of Starting Compounds" section, the following compounds are prepared:

4,6α,21,21-tetrafluoro-9α,11β-dichloro-16α-methyl-17α-hydroxypregna-1,4-diene-3,20-dione;

4,6α-difluoro-9α,11β,21,21-tetrachloro-16α-methyl-17α-hydroxypregna-1,4-diene-3,20-dione;

4,6α-difluoro-9α,11β,21-trichloro-16α-methyl-17α-hydroxypregna-1,4-diene-3,20-dione;

4,6α-difluoro-9α,11β-dichloro-16α-methyl-17α-hydroxy-21,21-dimethoxypregna-1,4-diene-3,20-dione;

4,6α-difluoro-9α,11β-dichloro-16α-methyl-17α,21-hydroxypregna-1,4-diene-3,20-dione;

4,6α-difluoro-9α,11β-dichloro-16α-methyl-17α-hydroxy-21-acetoxy-1,4-diene-3,20-dione; and the like.

## Example 13

This example sets forth a process for preparing 4,9α,11β-trichloro-6α-fluoro-11β,17α-dihydroxy-16α-methylpregna-1,4-diene-3,20-diones (which are substituted at the 21 position similarly to Example 12) according to the reaction sequence of Example 12 wherein $X^1$ and $X^3$ are chloro, $X^2$ is fluoro and $X^4$ is $=C{<}^{Cl}_{H}$ .

A. By following in principle the procedure of Example 12, Part A but substituting dichlorohydantoin for $ClO_3F$, 4,9α,11β-trichloro-6α,21-difluoro-16α-methyl-17α-hydroxypregna-1,4-diene-3,20-dione is obtained.

B. By following in principle the procedure of Part A of this example, but substituting an appropriate starting material according to the principles set forth in the "Preparation of Starting Compounds" section, the following compounds are obtained:

4,9α,11β-trichloro-6α,21,21-trifluoro-16α-methyl-17α-hydroxypregna-1,4-diene-3,20-dione;

4,9α,11β-trichloro-6α-fluoro-16α-methyl-17α-hydroxy-21-bromopregna-1,4-diene-3,20-dione;

4,9α,11β,21,21-pentachloro-6α-fluoro-16α-methyl-17α-hydroxypregna-1,4-diene-3,20-dione;

4,9α,11β-trichloro-6α-fluoro-16α-methyl-17α-hydroxy-21,21-dimethoxypregna-1,4-diene-3,20-dione;

4,9α,11β-trichloro-6α-fluoro-16α-methyl-17α,21-dihydroxypregna-1,4-diene-3,20-dione;

4,9α,11β-trichloro-6α-difluoro-16α-methyl-17α-hydroxy-21-acetoxypregna-1,4-diene-3,20-dione; and the like.

Example 14

This example sets forth a process for preparing 4,9α-difluoro-6α-chloro-11β,17α-dihydroxy-16α-methyl-pregna-1,4-diene-3,20-dione (which are substituted at the 21 position similarly to Example 11) according to the reaction sequence of Example 12 wherein $X^1$ and $X^3$ are fluoro, $X^2$ is chloro and $X^4$ is $=C\genfrac{}{}{0pt}{}{OH}{H}$.

A. 4,9α-Difluoro-6α-dichloro-11β,17α,21-trihydroxy-16α-methylpregna-1,4-diene-3,20-dione is prepared by following in principle the procedure set forth in Example 11, Part A, but substituting 6α-chloro-9α-fluoro-11β,17α,21-trihydroxy-16α-methylpregna-1,4-diene-3,20-dione for flumethasone.

B. By substituting other appropriate starting materials in the process of Part A of this example other compounds of this invention are prepared such as

4,9α-difluoro-6α,21-dichloro-11β,17α-dihydroxy-16α-methylpregna-1,4-diene-3,20-dione;

4,9α-difluoro-6α-chloro-11β,17α-dihydroxy-16α-methyl-21,21-dimethoxypregna-1,4-diene-3,20-dione; and the like.

Example 15

By following in principle the procedures of Example 9 where appropriate, 4-fluoro- or 4-chloro-16β-methyl-pregna-4-ene-3,20-diones are prepared according to the following reaction sequence:

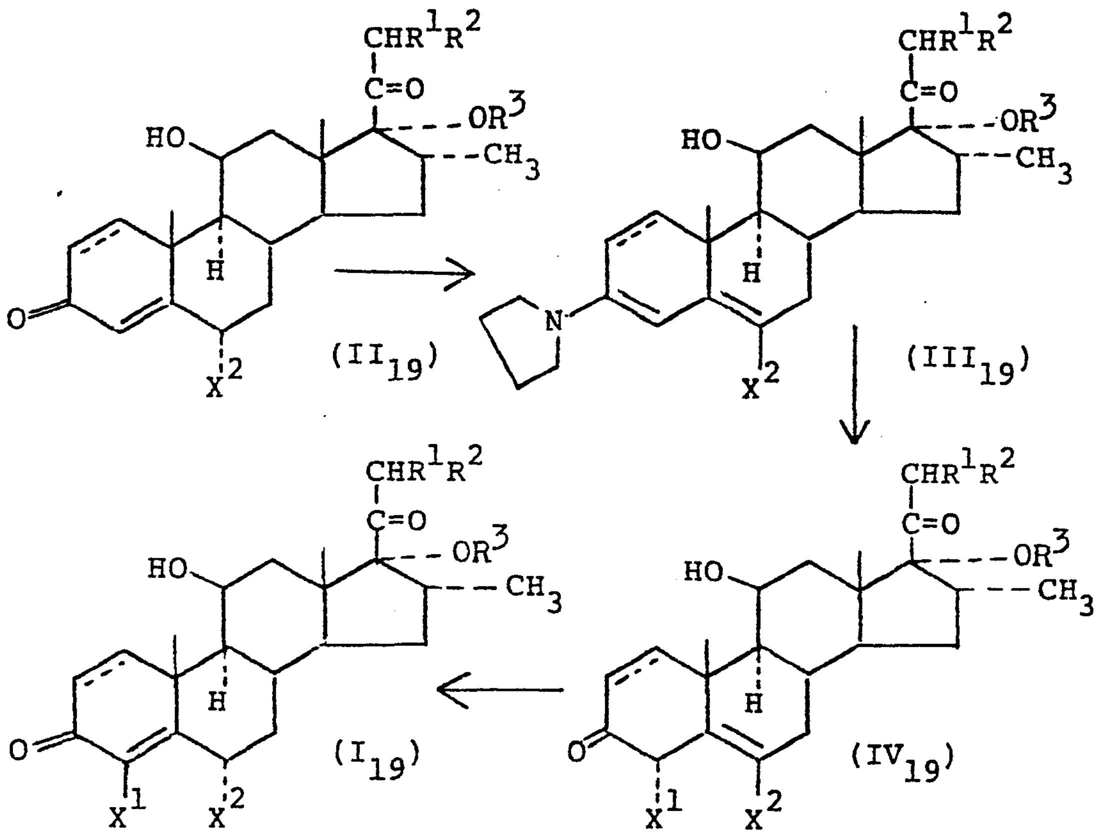

($II_{19}$) ($III_{19}$) ($I_{19}$) ($IV_{19}$)

wherein $X^1$ is fluoro or chloro and $X^2$ is fluoro, chloro or hydrogen, $R^1$ is hydrogen, fluoro, chloro, hydroxy or methoxy, $OR^3$ is OH or alkanoyloxy of 2 to 6 carbon atoms, and $R^2$ is fluoro, chloro, hydroxy or alkanoyloxy of 2-6 carbons when $R^1$ is hydrogen or $R^2$ is the same as $R^1$ when $R^1$ is fluoro, chloro, or methoxy, e.g.

4,6α-difluoro-11β-hydroxy-16β-methyl-17α-propionyloxy-21-chloropregna-1,4-diene-3,20-dione;

4-fluoro-11β-hydroxy-16β-methyl-17α-hexanoyloxy-21-chloropregna-1,4-diene-3,20-dione;

4-chloro-6α-fluoro-11β-hydroxy-16β-methyl-17α-propionyloxy-21-acetoxypregna-1,4-diene-3,20-dione, and the like.

## EXAMPLE 16

By following in principle the procedures set forth in Example 11 but substituting the corresponding

16β-methyl steroid starting material for the 16α-methyl steroid starting material, the corresponding 16β-methyl steroids of this invention are obtained such as 4,9α-difluoro-11β,17α-dihydroxy-16β-methyl-21-acetoxypregna-1,4-diene-3,20-dione, m.p. 130-134°C (semi-crystalline solid from ethanol-water);

4,6α-difluoro-11β,17α-dihydroxy-16β-methyl-21-acetoxypregna-1,4-diene-3,20-dione, m.p. 138-148°C (semi-crystalline solid from acetone-hexane);

4,6α,9α-trifluoro-11β,17α-dihydroxy-16β-methyl-21-acetoxypregna-1,4-diene-3,20-dione, m.p. 203-205°C; and the like.

Example 17

This example sets forth a process for preparing the 21,21-dihydroxy compounds of this invention from the corresponding 21-monohydroxy compound prepared according to Examples 1 or 11.

A solution of 0.68 g of cupric acetate hydrate in 40 ml methanol is added to a slurry of 12.0 g of 4,6α,9α-trifluoro-11β,21-dihydroxy-16α,17α-isopropylidenedioxypregna-1,4-diene-3,20-dione is 130 ml of dry methanol. Air is then sparged through the mixture for 2 hours. Thereafter, the mixture is evaporated to dryness and the residue is taken up in ethyl acetate and washed with water and then with a dilute aqueous solution of potassium bicarbonate and then again with water. The solution is evaporated to dryness and the resulting residue is then dissolved in acetone. The acetone solution is diluted wih a substantial valume of water whereupon the resulting precipitate is filtered and dried under vacuum to yield 4,6α,9α-trifluoro-11β,21,21-trihydroxy-16α,17α-isopropylidenedioxypregna-1,4-diene-3,20-dione.

Similarly, by following in principle the procedure of this Example but substituting other appropriate

starting materials for 4,6α,9α-trifluoro-11β,21-trihydroxy-16α,17α-isopropylidenedioxypregna-1,4-diene-3,20-dione the following compounds are prepared:

4,6α-difluoro-9α,11β-dichloro-21,21-dihydroxy-16α,17α-isopropylidenedioxypregna-1,4-diene-3,20-dione;

4-chloro-6α,9α-difluoro-11β,21,21-trihydroxy-16α,17α-isopropylidenedioxypregna-1,4-diene-3,20-dione;

4α-fluoro-11β,21,21-trihydroxy-16α,17α-isopropylidenedioxypregna-1,4-diene-3,20-dione

4,9α-difluoro-11β,21,21-dihydroxy-16α,17α-isopropylidenedioxypregna-1,4-diene-3,20-dione;

4-fluoro-6α,9α,11β-trichloro-21,21-dihydroxy-16α,17α-isopropylidenedioxypregna-1,4-diene-3,20-dione;

4,6α,9α-trifluoro-11β,17α,21,21-tetrahydroxy-16β-methylpregna-1,4-diene-3,20-dione;

4-chloro-6α,9α-difluoro-11β,17α-21,21-tetrahydroxy-16β-methylpregna-1,4-diene-3,20-dione;

4,9α,11β-trichloro-6α-fluoro-17α,21,21-trihydroxy-16β-methylpregna-1,4-diene-3,20-dione;

4,9α-difloro-11β,17α,21,21-tetrahydroxy-16α-methylpregna-1,4-diene-3,20-dione; and the like.

## Example 18

This example sets forth a process for converting the 17α-hydroxy substituent to a 17α-alkanoyloxy substituent, more particularly 17α-propionyloxy.

A. Three hundred milligrams (mg) of 4,6α,9α-trifluoro-11β,17α,21-trihydroxy-16α-methylpregna-1,4-diene-3,20-dione, prepared according to the process of Example 11, is added to a 50 milliliter (ml) flask along with 5 ml of triethyl orthopropionate and a few mg of dried p-toluene sulfonic acid (pTSA). The mixture is stirred for 1 hour under nitrogen at 120°C in a water bath until the reaction is complete. Fifty ml of ethyl acetate along with 100 ml of water are added to the

reaction mixture. After separating the water from the resulting organic phase, the latter is washed with three, 50 ml portions of water. The resulting organic mixture is dried over sodium sulfate, stripped and vacuum dried to give a yellow oil. The material is placed on a 0.75 millimeter (mm) by 1 meter (m) TLC place and developed three times with a 3% methanol:97% chloroform solvent mixture followed by extraction and crystallization from acetone-hexane to ultimately give about 185 mg of 4,6α,9α-trifluoro-11α,17α,21-trihydroxy-16α-methylpregna-1,4-diene-3,20-dione 17,21-ethyl orthopropionate.

B. One hundred eighty mg of 4,6α,9α-trifluoro-11β,17α,21-trihydroxy-16α-methylpregna-1,4-diene-3,20-dione 17,21-ethyl orthopropionate as prepared according to Part A, 6 ml of methanol and 1 ml of potassium hydrogen phosphate buffer (pH 3.1) are added to a 50 ml flask. The mixture is stirred overnight (approximately 17 hours) at room temperature which reults in about 70% completion of the reaction. An additional 30 ml of methanol and 5 ml of potassium hydrogen phosphate buffer are added and the reaction is heated to 60°C on a hot water bath for about 1 hour to bring the reaction to completion. The reaction mixture is partitioned between ethyl acetate and water and the ethyl acetate is removed by vacuum evaporation to give an oil which is then chromatographed on a 0.75 mm x 1 m TLC plate by developing two times with 3% methanol in chloroform to give 130 mg of 4,6α,9α-trifluoro-11β,17α,21-trihydroxy-16α-methylpregna-1,4-diene-3,20-dione 17-propionate.

C. Similarly, by following the above procedure in Part A but substituting for triethyl orthopropionate other orthoesters such as trimethyl orthoformate, triethyl orthoformate, tributyl orthoformate, trimethyl orthoacetate, triethyl orthoacetate, tripropyl orthoacetate, trihexyl orthoacetate, trimethyl ortho-

propionate, trimethyl orthobutyrate, tripropyl orthohexanoate, or trimethyl orthovalerate, and the corresponding 16β-methyl steroid for the 16α-methyl steroid other corresponding 17,21-orthoesters are obtained. These in turn are transformed to the 17-esters according to the procedure of Part B as shown in the following table:

Table 1

| This 17,21-orthoester | gives this 17-ester |
|---|---|
| 4,6α,9-fluoro-11β,17α,21-trihydroxy-16β-methylpregna-1,4-diene-3,20-dione 17,21-methyl orthoacetate; | 17-acetate |
| 4,6α,9α-fluoro-11β,17α,21-trihydroxy 16β-methylpregna-1,4-diene-3,21-dione 17,21-ethyl orthobutyrate | 17-butyrate |
| 4,6α,9α-fluoro-11β,17α,21-trihydroxy 16α-methylpregna-1,4-diene-3,21-dione 17,21-propyl orthohexanoate; | 17-hexanoate |
| 4,6α,9α-fluoro-11β,17α,21-trihydroxy 16α-methylpregna-1,4-diene-3,21-dione 17,21-ethyl orthovalerate | 17-valerate |

Similarly, by following in principle the procedure of Part A but substituting other appropriate steroids of this invention made according to Examples 12-22 for 4,6α,9α-trifluoro-11β,17α,21-trihydroxy-16α-methylpregna-1,4-diene-3,20-dione, other 17 esters of this invention are prepared.

Example 19

This example sets forth a process for making the $\Delta^4$ steroids of this invention.

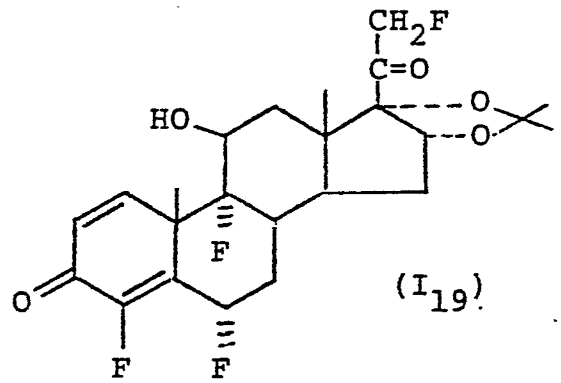

$(I_{19})$

A solution of 25 mg of tris-(triphenylphosphine) chlororhodium in 6 ml of benzene and 15 ml of ethanol is stirred under hydrogen for 60 minutes. 4,6α,9α,21,21-tetrafluoro-11β-hydroxy-16α,17α-isopropylidenedioxy-pregna-1,4-diene-3,20-dione (244 mg) represented by Formula $(I_{23})$, is added and the resulting solution is stirred under hydrogen at room temperature at atmospheric pressure. After hydrogen uptake is complete, the solution is evaporated to dryness and the residue taken up in a mixture of petroleum ether and methylene choride. The pure product is isolated by column chromatography on silica gel to give 4,6α,9α,21-tetrafluoro-11β-hydroxy-16α,17α-isopropylidenedioxypregn-4-ene-3,20-dione.

Similarly, by substituting other $\Delta^{1,4}$ steroids of this invention made according to Examples 1-22 for the compound of formula $(I_{23})$, other corresponding $\Delta^4$ steroids are prepared such as 4-chloro-6α-fluoro-11β-hydroxy-16α-methyl-17α-propionyloxy-21-acetoxypregna-1,4-diene-3,20-dione.

Example 20

This example sets forth a process for preparing an 11-keto compound of this invention by oxidizing any of the 11β-hydroxy steroids set forth in Examples 1-19.

One g of 4,6α,9α,21-tetrafluoro-11β-hydroxy-16α,17α-isopropylidenedioxypregna-1,4-diene-3,20-dione is dissolved in 50 ml of acetone and treated at room temperature with Jones reagent (chromic anhydride in dilute sulfuric acid) dropwise until TLC indicates the absence of starting material. The mixture is treated with five drops of isopropyl alcohol to destroy any excess of reagent, then diluted with 50 ml of water and the mixture concentrated under vacuum under reduced pressure to give a crystalline material, namely 4,6α,9α,21-tetrafluoro-16α,17α-isopropylidenedioxypregna-1,4-diene-3,11,20-trione.

Example 21

This example sets forth a process for converting a $\Delta^4$-steroid to a $\Delta^{1,4}$ steroid.

A mixture of 0.5 g of 4,6α-difluoro-11β,21-dihydroxy-16α,17α-isopropylidenedioxypregn-4-ene-3,20-dione, 10 ml of dioxane and 0.35 g. of 2,3-dichloro-5,6-dicyano-1,4-benzoquinone is refluxed for 10 hours. The mixture is then cooled, filtered and evaporated to dryness. The residue is dissolved in acetone and this solution is then filtered through 10 g of alumina and concentrated to yield 4,6α-difluoro-11β,21-dihydroxy-16α,17α-isopropylidenedioxypregna-1,4-diene-3,20-dione which is further purified by recrystallization from acetone:hexane.

Example 22

This example sets forth an alternative route to the 16β-methyl steroid of this invention.

A. Ten (10) g of 6α-fluoro-16β-methyl-17α,21-diacetoxypregna-1,4,9(11)-triene-3,20-dione in 110 ml of dioxane (A.R.) plus 2.2 mls of a solution of 4.4 ml 70% $HClO_4$ in 200 mls of $H_2O$, treated with 4 g of dibromantoin in the dark at R.T. for a period of one hour, when TLC in 50% ethyl acetate/50% hexane shows the reaction to be complete. The reaction mixture is precipitated in 2 l of water, stirred for 10 minutes and the crystalline precipitate collected by filtration, washed with water, and air dried to give 11.4 g of 6α-fluoro-9α-bromo-11β-hydroxy-16β-methyl-17α,21-diacetoxy pregna-1,4-diene-3,20-dione. This bromohydrin (19.1 g) is mixed with 286 ml of methyl orthoformate, 96 ml of anhydrous methanol and 1.9 ml of fuming sulfuric acid and heated on a water bath at 50-55°C for 15 minutes. The mixture is treated with 15 ml of pyridine and poured into 300 ml of water, separated and washed three times with water. The resulting mixture is dried over anhydrous $Na_2SO_4$, filtered and concentrated under high vacuum to a foam which is left in crushed dry ice for 16 hours to give the 11β-orthoester of 3-methoxy-6α-fluoro-9α-bromo-16β-methyl-17α,21-diacetoxypregna-1,3,5(6)-triene-20-one. The orthoester so obtained is dissolved in 300 ml of a mixture of 80% THF/20% water and treated at room temperature with a slow stream of $ClO_3F$ until no more starting material is detected by TLC analysis. The mixture is diluted with water and the organic solvent eliminated under reduced pressure (high vacuum) at 50-55°C. The mixture is diluted with water up to 2 liters and kept in the refrigerator for 20 hours. The resulting precipitate is filtered and dried. One (1) g of crude reaction mixture is dissolved in about 20 ml of

methylene dichloride (MDC) and filtered through a 10 g column of silica with 100% MDC. The column is eluted with 1.2 liters of MDC, then with 2% ethyl acetate/98% MDC. The homgeneous fractions (containing small amounts of negative and positive polar impurities are concentrated to dryness under high vacuum. NMR analysis of the negative polar product eluted indicates that the product is 4,6α-difluoro-9α-bromo-11β-hydroxy-16β-methyl-17α,21-diacetoxypregna-1,5(6)-diene-3,20-dione.

The resulting product is mixed with tin tributylhydride in tetrahydrofuran at room temperature to eliminate the 9α-bromine and form 4,6α-difluoro-11β-hydroxy-16β-methyl-17α,21-diacetoxypregna-1,4(6)-3,20-dione. (The reaction may be accelerated by adding a small amount of a free radical and refluxing.)

The resulting product is stirred with methanol containing anhydrous potassium carbonate under nitrogen at atmospheric pressure and ambient temperature until TLC shows the reaction is complete. The reaction mixture is diluted with methanol and glacial acetic acid and concentrated under reduced pressure to a small volume. The crystalline precipitate which forms is collected by filtration and washed with methanol and water to give 4,6α-difluoro-11β,17α,21-trihydroxy-16β-methylpregna-1,4-diene-3,20-dione.

B. By following in principle the process set forth in Example 18 the 17α-hydroxy substituent can be converted to a 17α-alkanoyloxy substituent. Compounds prepared in this manner include 4,6α-difluoro-11β,21-dihydroxy-16β-methyl-17α-propionyloxypregna-1,4-diene-3,20-dione;

4,6α-difluoro-11β,21-dihydroxy-16β-methyl-17α-acetoxy pregna-1,4-diene-3,20-dione;

4,6α-difluoro-11β,21-dihydroxy-16β-methyl-17α-butyryloxypregna-1,4-diene-3,20-dione; and the like.

## Example 23 - Formulation

In this example a formulation is prepared of the following composition

| | % w/w |
|---|---|
| 4,6α,9α-trifluoro-11β-hydroxy-16α,17α-isopropylidenedioxypregna-1,4-diene 3,20-dione | 0.025 |
| Stearyl Alcohol | 30.0 |
| PEG 6000 | 5.0 |
| 1,2,6-Hexanetriol | 2.5 |
| Citric Acid Anhydrous, USP | 0.02 |
| Propylene Glycol, USP, q.s. | 100.0 |

The steroid is dissolved in 624.8 grams of propylene glcyol at 90-95°C. The latter is then mixed with the other ingredients at 80-85°C to give the desired formulation.

## Example 24 - $LD_{50}$

Six Swiss-Webster mice (Simonsen) each weighing about 25 grams, were injected subcutaneously with a solution of 4,6α,9α-trifluoro-11β-hydroxy-16α,17α-isopropylidenedioxypregna-1,4-diene-3,20-dione in carbomethoxycellulose having a concentration of 10 ml/kg. The dosage was 25 mg/kg or about .625 mg/mouse. The mice were observed daily for mortality for 21 days. No mice died. The $LD_{50}$ is, therefore, more than 25 mg/kg.

## Example 25 - Biological Activity

### Anti-inflammatory Activity

This example sets forth the results of the topical anti-inflammatory activity of compounds of this invention. The topical anti-inflammatory activity

potential for each compound was asayed using a modified Stoughton/McKenzie vaso-constriction assay in humans, i.e., McKenzie, W.W. and Stoughton, R.B. "Method for Comparing Percutaneous Absorption of Steroids" Arch. Dermat. 86, 608 (1962).

Eight normal adult human subjects were treated on four sites on each forearm by topical administration with alcoholic solutions containing 1 X $10^{-5}$ and 1 X $10^{-6}$ g/ml of each of the compounds to provide 64 total test sites for each compound in a series (32 for each concentration). Areas of the subjects' forearms were outlined by a rubber stamp grid coated with silicone grease, and 10 microliters of each solution were applied per 7x7 mm square site. After the preparations dried, the areas on each forearm are covered with Saran$^{R}$ wrap and the margins sealed with tape. The occlusive wrap is removed after 18 hours. Twenty-four hours after application, the presence or absence of vasoconstriction is noted by visual examination by two independent observers, and expressed as the number of sites responding (vasoconstriction) and is calculated as a percentage of the total number of sites. Also, the intensity of the vasoconstriction is scored on a 0, 1, 2 scale, 2 being the most intense reaction. Both scores are used in the constructing dose-response graphs according to methods set forth in an article by Place, V.A. et al entitled "Precise Evaluation of Topically Applied Corticosteroid Potency", Arch Derm, 101, 531-537 (1970). The activity is determined relative to fluocinolone acetonide (FA). If the compound exhibits activity in the range of 0.1 times the activity of FA, it is an active compound.

Thymolytic Activity

The compounds to be tested, including a hydrocortisone standard, were prepared in three or more concentrations by suspension in a sodium carboxymethylcellulose vehicle. Animals received the test materials by subcutaneous injection of 0.5 ml of the suspension on each of three successive days. Four hours following the final injection, the rats were sacrificed and the thymus gland of each animal removed and weighed and a determination of activity relative to hydrocortisone (HC) obtained. HC has low thymolytic activity.

Generally, the compounds of this invention show high anti-inflammatory activity but low thymolytic activity, thus exhibiting a therapeutic advantage over compounds of the art.

The following table sets forth the results wherein $X^1$, $X^3$, $X^4$, $R^1$ and $R^2$ refer to the formula given.

TABLE I

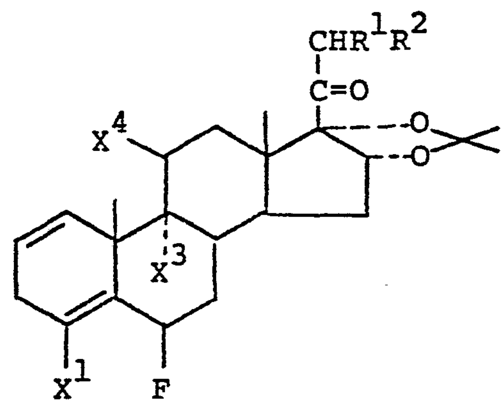

| 16α,17α-acetonides | | | | | Anti-Inflammatory | Thymolytic |
|---|---|---|---|---|---|---|
| $X^1$ | $X^3$ | $X^4$ | $R^1$ | $R^2$ | | |
| F | F | OH | H | Cl | 1.5 X FA | 10 X HC |
| F | F | OH | $CH_3O$ | $CH_3O$ | 0.2 X FA | 2 X HC |
| F | F | OH | H | F | 0.1 X FA | 4 X HC |
| Cl | F | OH | H | Cl | 0.3 X FA | 8 X HC |
| F | Cl | Cl | H | Cl | 1.8 X FA | 2 X HC |

CLAIMS:

1. A compound chosen from those represented by the formula

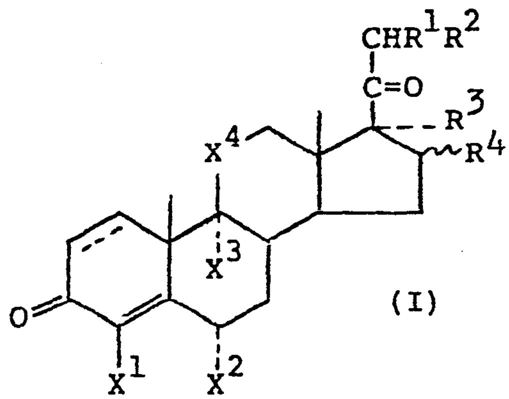

(I)

wherein

$X^1$ is fluoro or chloro;

$X^2$ is hydrogen, fluoro or chloro;

$X^3$ is hydrogen, fluoro, chloro or bromo;

$X^4$ is =C=O or 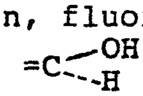 or may be 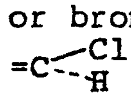 when $X^3$ is chloro;

$R^1$ is hydrogen, fluoro, chloro, bromo, hydroxy or methoxy;

$R^2$ is fluoro, chloro, hydroxy or alkanoyloxy of 2 to 6 carbon atoms when $R^1$ is hydrogen or $R^2$ is the same as $R^1$ when $R^1$ is fluoro, chloro, hydroxy or methoxy;

$R^3$ is hydroxy or alkanoyloxy of 2 to 6 carbon atoms when $R^4$ is α-methyl or β-methyl or $R^3$ and $R^4$ together are 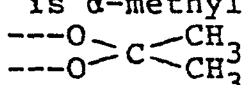 ; and

the solid and broken lines between the 1- and 2-positions in the A ring of the steroid nucleus represents a single or a double bond.

2. The compound of Claim 1 wherein $X^2$ is hydrogen or fluoro, $X^3$ is hydrogen or fluoro; $X^4$ is 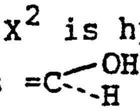 ; $R^1$ is hydrogen, hydroxy or methoxy; $R^2$ is hydroxy,

fluoro, chloro or acetoxy when $R^1$ is hydrogen or $R^2$ is the same as $R^1$ when $R^1$ is hydroxy or methoxy; and $R^3$ and $R^4$ together are 16α,17α-isopropylidenedioxy.

3. The compound of Claim 1 wherein $X^1$ is fluoro or chloro; $X^2$ is hydrogen or fluoro; $X^3$ is fluoro or hydrogen; $X^4$ is $=C{<}^{OH}_{H}$; $R^1$ is hydrogen, hydroxy or methoxy; $R^2$ is fluoro, chloro, hydroxy or acetoxy when $R^1$ is hydrogen or $R^2$ is the same as $R^1$ when $R^1$ is hydrogen or methoxy; $R^3$ is hydroxy or alkanoyloxy of 2-6 carbon atoms; and $R^4$ is α-methyl or β-methyl.

4. The compound of Claim 3 wherein $R^1$ is hydrogen; $R^2$ is fluoro, chloro, hydroxy or acetoxy and $R^4$ is α-methyl.

5. A topical anti-inflammatory pharmaceutical composition which comprises a therapeutically effective amount of a compound chosen from those of Claim 1 in combination with at least on suitable pharmaceutical excipient.

6. A compound of claim 1 for use in relieving a topical inflammatory condition in a mammal.

7. A process for preparing the compound of Claim 1 wherein $R^3$ is alkanoyloxy of 2 to 6 carbon atoms, $R^4$ is α-methyl or β-methyl, and $X^1$, $X^2$, $X^3$, $X^4$, $R^1$, $R^2$ and the broken line between C-1 and C-2 are as defined in Claim 1, which process comprises esterifying the corresponding 17α-hydroxy compound.

0004766

8. The process for preparing the compound of Claim 1, which process comprises contacting a compound represented by the formula

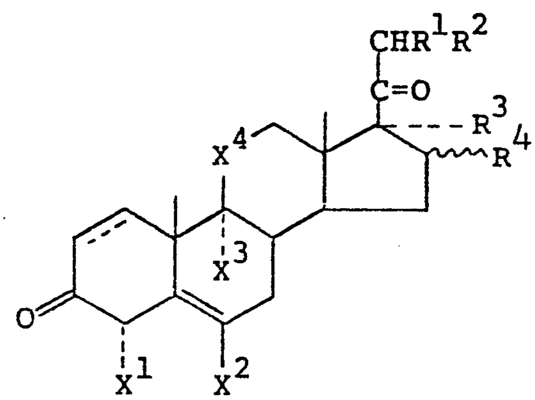

wherein $R^1$, $R^2$, $R^3$, $R^4$, $X^1$, $X^2$, $X^3$, $X^4$ and the broken line between C-1 and C-2 are as defined in Claim 1 with a base.